(19) Europäisches Patentamt

European Patent Office

Office européen des brevets

(11) EP 0 626 838 B1

# (12) EUROPEAN PATENT SPECIFICATION

(45) Date of publication and mention
of the grant of the patent:
**08.12.1999 Bulletin 1999/49**

(21) Application number: **92914061.4**

(22) Date of filing: **18.02.1992**

(51) Int. Cl.$^6$: **A61F 13/46**, B32B 3/24,
B29C 59/06

(86) International application number:
**PCT/US92/01056**

(87) International publication number:
**WO 93/15701 (19.08.1993 Gazette 1993/20)**

(54) **TEXTILE-LIKE APERTURED PLASTIC FILMS**

TEXTILÄHNLICHE, GELOCHTE PLASTIKFOLIE

FILMS PLASTIQUES POREUX RESSEMBLANT AU TISSU

(84) Designated Contracting States:
**AT BE CH DE DK ES FR GB GR IT LI LU MC NL
SE**

(43) Date of publication of application:
**07.12.1994 Bulletin 1994/49**

(73) Proprietor: **McNEIL-PPC, INC.
New Brunswick, New Jersey 08933 (US)**

(72) Inventors:
• **TURI, Mordechai
  Princeton Junction, NJ 08550 (US)**
• **DEROSSETT, Edmund, Z.
  Mercerville, NJ 08619 (US)**

• **YANG, Ching-Yun, M.
  Princeton Junction, NJ 08550 (US)**

(74) Representative:
**Mercer, Christopher Paul
Carpmaels & Ransford
43, Bloomsbury Square
London WC1A 2RA (GB)**

(56) References cited:
| | |
|---|---|
| EP-A- 0 018 020 | EP-A- 0 165 807 |
| EP-A- 0 532 937 | US-A- 2 487 240 |
| US-A- 3 989 867 | US-A- 4 262 049 |
| US-A- 4 324 246 | US-A- 4 629 643 |
| US-A- 4 735 843 | US-A- 4 839 216 |

EP 0 626 838 B1

## Description

### Field of the Invention

The present invention relates generally to apertured films and more particularly to apertured plastic films comprising a plurality of micro-holes defined by a network of fiber-like elements or micro-strips of drawn plastic material. The invention also relates to methods and apparatus for making such apertured films and to products comprising such apertured films.

### Background of the Invention

Nonwoven fabrics have been used for a wide variety of applications for at least fifty years. Nonwoven fabrics are textile-like materials produced directly from a web of fibers so as to eliminate the many time consuming steps involved in converting staple length fibers into woven or knitted goods. In one method of making a nonwoven fabric, a web of fibers is produced, e.g. by carding or air laying techniques, and the fibrous web is then strengthened by the application thereto of a polymeric binding agent. In another method of making a nonwoven, the fibrous web is subjected to fluid forces which serve to entangle the fibers, thus providing strength to the finished material. Nonwoven fabrics are inherently porous structures, i.e. they comprise openings or pores allowing for the passage of fluids such as air and water or aqueous solutions. In addition, nonwoven fabrics may be tailored so as to have good softness, drapeability, and tactile impression. Owing to their desirable characteristics, nonwovens have been employed as facing materials for absorbent products such as disposable diapers, sanitary napkins, incontinent devices, wound dressings and the like.

More recently, efforts have been made to produce porous or liquid-permeable facing materials for absorbent products by using plastic films as the starting materials. For example, it is known to produce an apertured plastic film by placing a heated thermoplastic sheet material on a patterned perforated surface and applying a vacuum thereto. The vacuum pulls the softened sheet material through the perforations, thereby causing the film to rupture and form apertures.

U.S. 3,929,135 to Thompson et al discloses perforated topsheet materials for absorptive devices such as sanitary napkins, incontinent pads, bandages and the like. These topsheet materials are constructed from liquid impervious materials such as low density polyethylene and comprise a plurality of tapered capillaries each of which has a base opening in the plane of the top sheet and an apex opening which is remote from the plane of the top sheet. The tapered capillaries disclosed by Thompson et al. are preferably provided in the form of a frustum of a conical surface and have an angle of taper of from about 10° to 60°.

U.S. 4,324,246 to Mullane discloses an apertured formed film having a caliper of less than about 0.075 cm (0.030 inch), an open area of at least 35% and a plurality of apertures at least 75% of which have an equivalent hydraulic diameter (EHD) of at least, 0.064 cm (0.025 inch). The apertured formed film disclosed by Mullane et al. is useful as a topsheet for disposable absorbent products of the type mentioned above.

U.S. 4,839,216 to Curro et al. discloses a debossed and perforated plastic material produced by providing a starting film on a perforated forming surface and applying an unconstrained liquid stream to the upper surface of the starting film. The liquid stream has sufficient force and mass flux to cause the film to be deformed toward the forming surface, such that the material acquires a substantial three-dimensional conformation, and to cause perforations to be created therein.

European Patent Application 0 304 617 in the name of Kao Corporation discloses a covering sheet for a sanitary article. The covering sheet comprises an opaque, hydrophobic film having land portions and recesses, said recesses being formed to have a bottom portion and side walls. The side walls have a slanting part which is provided with an opening such that the slanting part is not covered by the land portion. This patent states that the opening is always exposed to sight when it is looked down at.

U.S. 4,690,679 discloses an apertured film comprising a first layer of a first polymeric film and a second layer of a second polymeric film. Apertured films wherein the apertures have average equivalent circular diameters ranging from about 0.0254 cm (0.010 inches) to about 0.0762 cm (0.030 inches) are disclosed as being useful as covering materials for absorbent products.

U.S. 4,629,643 to Curro *et al.* discloses a microapertured polymeric web said to have improved tactile properties which is produced by impinging a jet of high pressure fluid on the exposed surface of a web of flat polymeric film supported on a fine mesh woven wire support member. The smooth flat film assumes the overall knuckle pattern of the woven wire support member and volcano-shaped microapertures form in the portions of the web coinciding with the interstices between the intersecting woven wire filaments.

European Patent Application 0 018 020 in the name of The Procter & Gamble Company discloses a resilient plastic web formed by debossing and optionally perforating a film in the image of a perforated laminate forming surface.

Other patents relating to apertured films and methods and apparatus for making the same include U.S.

3,632,269 to Doviak *et al.* and U.S. 4,381,326 to Kelly.

**Summary of the Invention**

According to a first aspect of the present invention there is provided an apertured film comprising a polymeric material having a plurality of micro-holes therein, characterised in that the polymeric material comprises a stretchable thermoplastic polymeric material and the micro-holes are defined by a network of fiber-like elements, wherein the thermoplastic polymeric material comprising said fiber-like elements, has been drawn, the said fiber-like elements having been drawn at least 100 percent as compared to adjacent regions of starting film which had not undergone aperturing during processing.

In accordance with one aspect of the present invention, there are provided apertured films which have the porosity, open area, softness, strength, hand, textile-like appearance, tactile impression, conformability and draping properties characteristic of many nonwoven fabrics made from fibrous webs, and which do not have the undesirable characteristics, such as stiffness, non-conformability and "plastic feel" often encountered in apertured plastic films of the prior art.

Apertured plastic films of the present invention comprise a plurality of micro-holes defined by a network of fiber-like elements or micro-strips of drawn plastic material. The micro-holes are for the most part irregular in shape, that is, they do not assume a clearly identifiable geometric configuration such as circular, square or oval. The micro-holes may be provided in a discontinuous pattern of distinct groups or clusters. The pattern of distinct groups or clusters of micro-holes may be either random or regular; in either instance the micro-holes in each distinct group or cluster may be randomly distributed therein. Furthermore, the micro-holes are non-uniform in size and have small equivalent hydraulic diameters (EHD).

The individual fiber-like elements in the network of fiber-like elements comprise drawn portions of the starting plastic film. These fiber-like elements, sometimes also referred to herein as "fibrils", cooperate with the above-mentioned micro-holes to provide the apertured film of the invention with a visual appearance reminiscent of nonwoven fabrics made from air-laid or carded webs of staple-length fibers or spun-bonded nonwovens. The fiber-like elements also provide the apertured films of the invention with good softness and drapeability. The fiber-like elements or fibrils have lengths ranging from about 0.013 cm (0.005 inch) to about 0.127 cm (0.05 inch); widths ranging from about 0.003 cm (0.001 inch) to about 0.089 cm (0.035 inch); and thickness ranging from about 0.0006 cm (0.00025 inch) to about 0.005 cm (0.002 inch).

Apertured films according to the present invention may have low open areas, ranging from about 1% to 15%. Such low open areas are advantageous in instances where the apertured film of the invention is used as a cover material for the absorbent core of products such as sanitary napkins and disposable diapers. In such cases, the low open area substantially reduces the tendency of fluids (e.g. menstrual fluid or urine) absorbed by the absorbent core to travel back through the apertured film to rewet its upper surface and contact the skin of the wearer. At the same time, the apertured films of the present invention, when suitably pigmented, e.g. with titanium dioxide, are very effective in masking stains on the surface of the absorbent core as a result of having been contacted by body exudates such as menstrual fluid and urine.

In certain embodiments, the apertured film of the invention further includes a plurality of secondary openings whose area is substantially larger than the area of the aforementioned micro-holes. It will be understood that the percent open area of the apertured film may be varied over a wide range by varying the number and/or size of the micro-holes and/or the secondary openings.

According to a second aspect of the present invention there is provided a method for forming an apertured film comprising a polymeric material having a plurality of micro-holes therein, said method comprising:

a) providing a starting film comprising a polymeric material and having an upper surface and a lower surface;

b) providing a backing member comprising localized support regions for supporting a film, recessed zones into which the film may be deformed by the application thereto of fluid forces; and means for allowing said applied fluid to be transported away from said backing member;

c) supporting said starting film on said backing member with the lower surface of said film being in contact with the support surface of said backing member and with the upper surface of said film facing away from said backing member;

d) directing a fluid force against the upper surface of said starting film in a zone of contact and at a pressure sufficient to cause the formation of a plurality of said micro-holes;

e) moving said film from said contact zone; and

f) removing said now-apertured film from said backing member,

characterised by:

(a) providing a starting film comprising a stretchable thermoplastic polymeric material; and

(d) directing a fluid force in the form of columnar streams to cause the formation of a plurality of micro-holes and to draw the thermoplastic polymeric material at least 100 per cent as compared to adjacent regions of starting film which had not undergone aperturing during processing so as to provide a network of drawn fiber-like elements defiring said micro-holes.

The apertured films of the present inventions are produced by directing controlled fluid forces against one surface of a relatively thin, stretchable plastic film while the film is supported on its other surface by a backing member. Backing members suitable for use in the practice of the present invention comprise localized support regions for supporting the film; recessed zones into which the film may be deformed by the application of the fluid forces thereto; and means for moving the applied fluid away from the backing member.

In one specific method for producing apertured films in accordance with the present invention, the backing member includes a plurality of spaced-apart, upwardly extending pyramids arranged in a predetermined pattern on one surface of the backing member. The pyramids have bases which are four-sided and the pyramids are oriented so that the side edges defining the base of the pyramids are disposed at an angle of about 60° with respect to the side edges of the backing member (said side edges of the backing member corresponding and being parallel to the machine direction of the film as it is disposed on said backing member). The plurality of pyramids in said specific pattern are arranged in rows running crosswise of the backing member and in columns running lengthwise of the backing member, with the pyramids in any given row being staggered or offset with respect to the pyramids in each of the two rows immediately adjacent said given row. The side walls of the plurality of pyramids define a first set of valleys and a second set of valleys, with each set of valleys running at an angle to the machine direction of the backing member. The two sets of valleys mutually intersect each other. The valleys in each set of valleys are arranged in parallel relationship with each other.

The backing member under discussion further comprises a plurality of circular openings for removal of the applied fluid. The openings are located in the aforementioned valleys between the pyramids.

In another specific method for producing apertured film in accordance with the present invention, the backing member for supporting the plastic film comprises a base portion and a plurality of continuous, upwardly extending, spaced-apart, arcuate ribs disposed in parallel relationship with respect to one another. The arcuate ribs, as seen in top plan view, are in the form of a sine-like wave. The backing member further comprises a plurality of valleys or depressed regions defined by adjacent pairs of arcuate ribs. A plurality of apertures are located in the depressed regions and run through the backing member from one major surface to the other.

In yet another specific method for producing apertured film in accordance with the present invention, the backing member comprises a base portion with a plurality of apertures running through the thickness thereof. In this embodiment of the backing member, which may be provided in flat plate or cylindrical form (as may the aforementioned backing members) there are no upstanding elements such as the aforementioned pyramids or sinusoidal-like ribs.

## Brief Description of the Drawings

The present invention will be more clearly understood by reference to the accompanying detailed description and the appended drawings in which:

FIG. 1 is a schematic perspective view of the upper surface of one embodiment of an apertured plastic film according to the present invention;

FIG. 2 is a photomicrograph of the upper surface of the apertured film illustrated schematically in FIG. 1 enlarged about 20 times;

FIG. 3 is a photomicrograph of the lower surface of the apertured film illustrated schematically in FIG. 1 enlarged about 20 times;

FIG. 4 is a photomicrograph of the upper surface of the apertured film illustrated schematically in FIG. 1 enlarged about 40 times;

FIG. 5 is a photomicrograph of the lower surface of the apertured film illustrated schematically in FIG. 1 enlarged about 40 times;

FIG. 6 is a schematic perspective view of the upper surface of a second embodiment of an apertured plastic film according to the present invention;

FIG. 7 is a photomicrograph of the upper surface of the apertured film illustrated schematically in FIG. 6 enlarged about 20 times;

FIG. 8 is a photomicrograph of the lower surface of the apertured film illustrated schematically in FIG. 6 enlarged about 20 times;

FIG. 9 is a photomicrograph of the upper surface of the apertured film illustrated schematically in FIG. 6 enlarged about 40 times;

FIG. 10 is a photomicrograph of the lower surface of the apertured film illustrated schematically in FIG. 6 enlarged about 40 times;

FIG. 11 is a schematic plan view of the upper surface of a third embodiment of an apertured plastic film according to the present invention;

FIG. 12 is a photomicrograph of the upper surface of the apertured film illustrated schematically in FIG. 11 enlarged about 20 times;

FIG. 13 is a photomicrograph of the lower surface of the apertured film illustrated schematically in FIG. 11 enlarged about 20 times;

FIG. 14 is a photomicrograph of the upper surface of the apertured film illustrated schematically in FIG. 11 enlarged about 40 times;

FIG. 15 is a photomicrograph of the lower surface of the apertured film illustrated schematically in FIG. 11 enlarged about 40 times;

FIG. 16 is a schematic view of apparatus for producing apertured films according to the present invention;

FIG. 17 is an exploded perspective view of a starting plastic film and a backing member on which the starting film is positioned for processing in accordance with the present invention;

FIG. 18 is a top plan view of the backing member shown in the lower portion of FIG. 17;

FIG. 19 is a cross-sectional view taken along line 19-19 of FIG. 18;

FIG. 20 is a block diagram showing the various steps of the process for producing apertured film according to the present invention;

FIG. 21 is a diagrammatic view of another type of apparatus for producing apertured films according to the present invention;

FIG. 22 is a diagrammatic view of a preferred apparatus for producing apertured films according to the present invention;

FIG. 23 is a top plan view of the backing member used to produce the apertured film shown in FIGS. 6-10;

FIG. 24 is a cross-sectional view taken along line 24-24 of FIG. 23;

FIG. 25 is a top plan view of the backing member used to produce the apertured film shown in FIGS. 11-15;

FIG. 26 is a cross-sectional view taken along line 26-26 of FIG. 25;

FIG. 27 is a perspective view of a sanitary napkin comprising an apertured film according to the present invention; and

FIG. 28 is a view, partially in section, taken along line 28-28 of FIG. 27.

## Description of the Preferred Embodiments

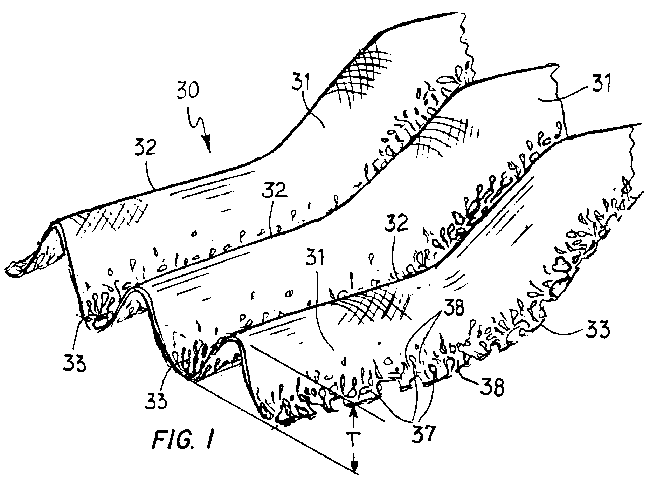

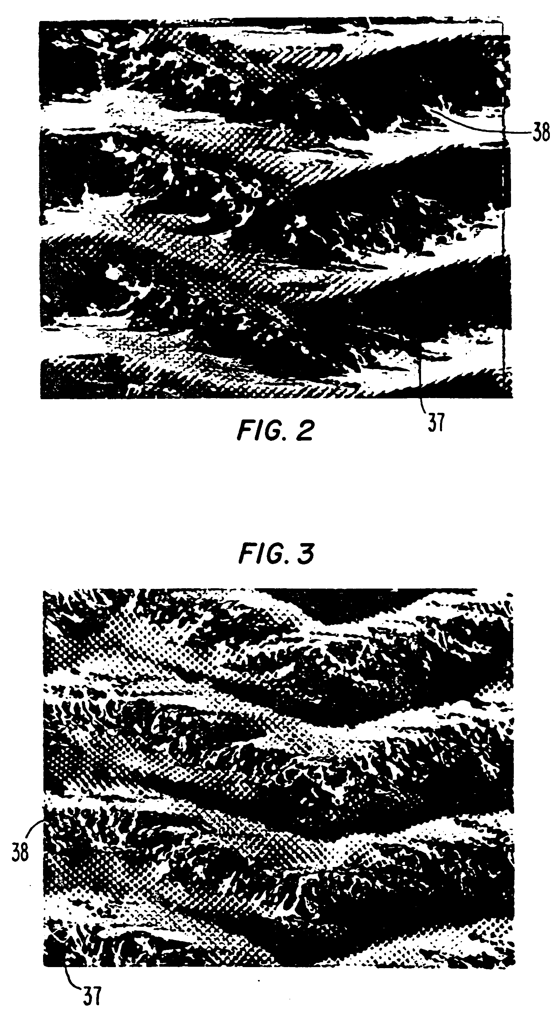

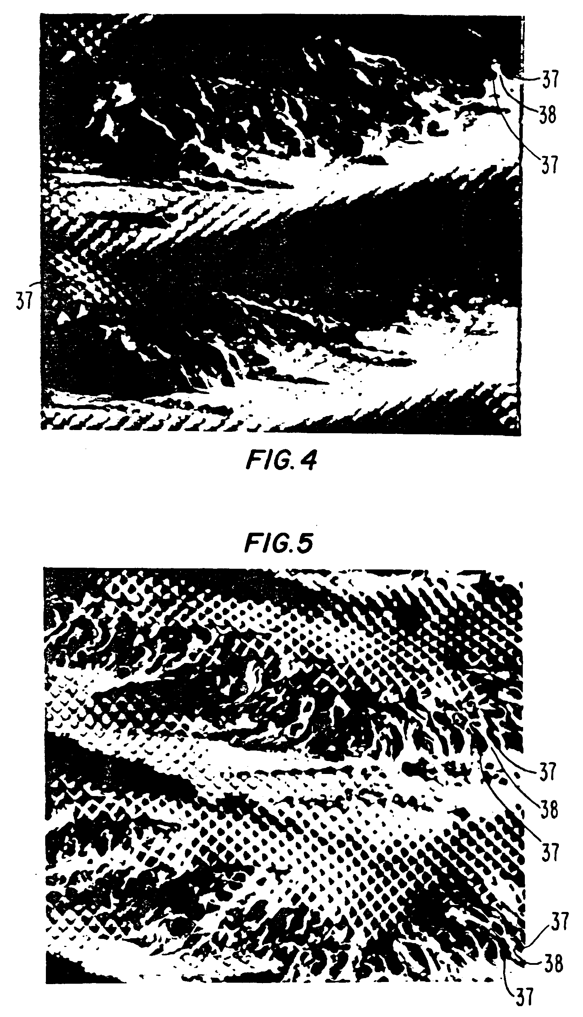

Referring now to FIGS. 1-5 of the drawings, there is shown one embodiment of an apertured plastic film in accordance with the present invention. Apertured plastic film 30 is made from a thin layer of embossed, stretchable thermoplastic polymeric material which, prior to processing, has a thickness which may conveniently range from about 0.0013 cm (0.0005 inch) to about 0.0127 cm (0.005 inch). Apertured plastic film 30 comprises angled, generally vertically oriented side walls 31 which meet on the upper surface of the film to form a series of generally parallel ridges 32. The angled side walls define a series of generally parallel valleys 33. As seen in plan view, both the ridges and the valleys have an arcuate, sinusoidal-like configuration. The valleys 33 and the lower reaches of side walls 31 adjacent those valleys comprise a multiplicity of micro-holes 37 defined by a network of very fine fiber-like elements 38. The bulk or thickness, T, of apertured film 30, i.e. the vertical distance from a valley 33 to a ridge 32 is approximately 0.05 cm (20 mils).

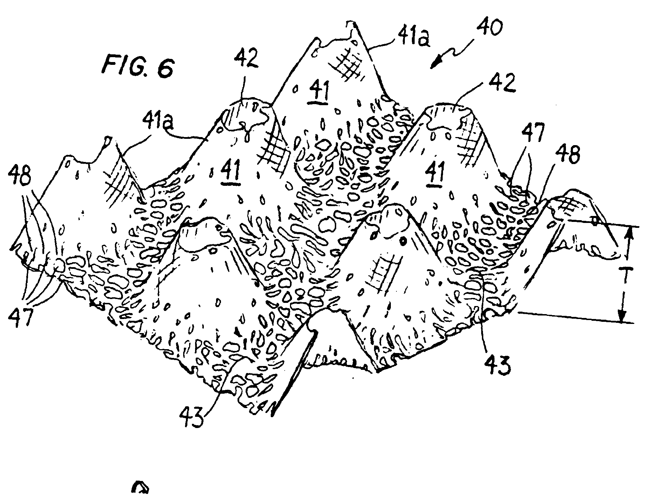

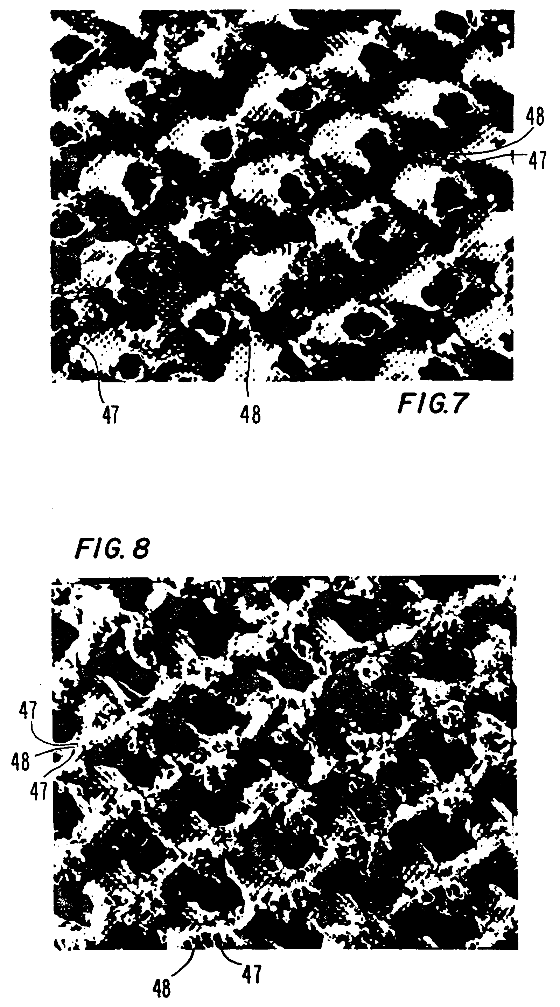

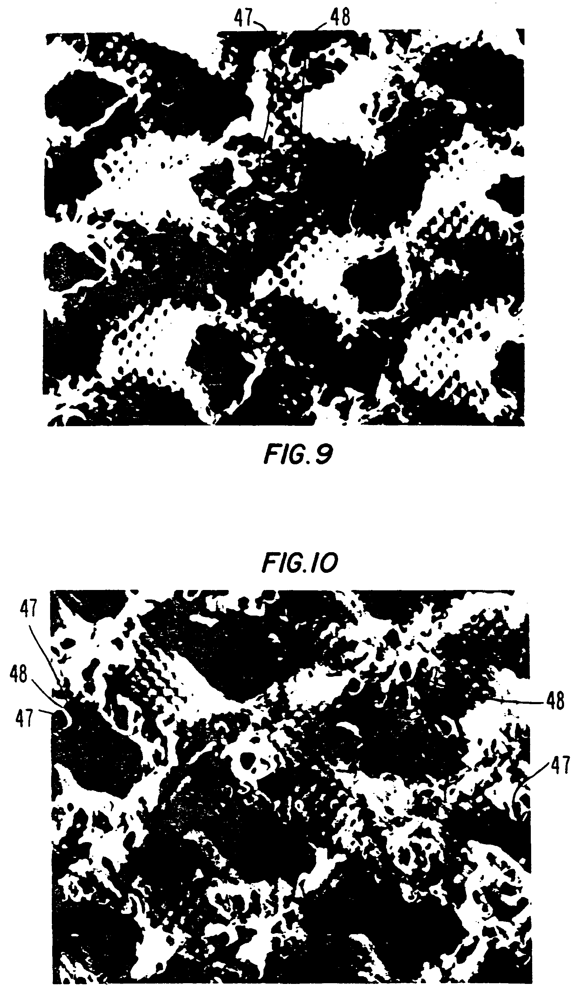

Turning to FIGS. 6-10 of the drawings, there is shown a second embodiment of an apertured plastic film in accordance with the present invention. Apertured film 40 is also made from a layer of embossed, stretchable, thermoplastic polymeric material whose thickness, prior to processing, is in the range of from about 0.0013 cm to about 0.0127 cm. Apertured film 40 comprises a plurality of generally vertically extending cone-like structures 41 whose sloping side walls 41a define valleys 43 therebetween. The valleys 43 and adjacent portions of the side walls of the cone-like structures comprise a large plurality of micro-holes 47 defined by a network of very fine fiber-like elements 48. Cone-like structures 41 comprise secondary openings 42 whose areas are substantially larger than the areas of micro-holes 47. The bulk or thickness, T, of the apertured plastic film, i.e. the perpendicular distance from a valley 43 to the top of cone-like structure 41 is approximately 0.064 cm (25 mils).

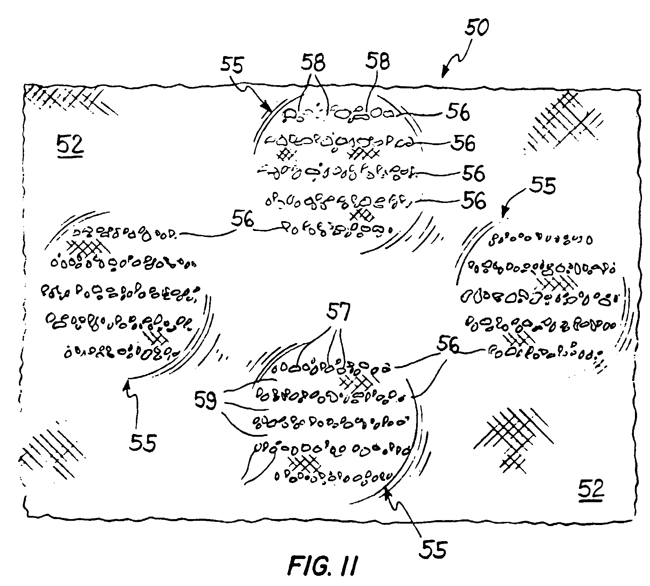

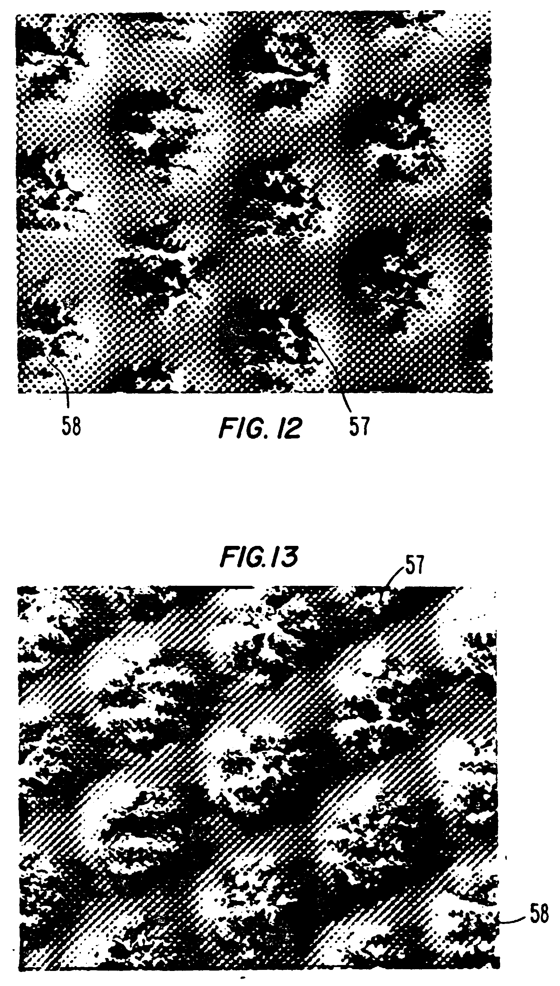

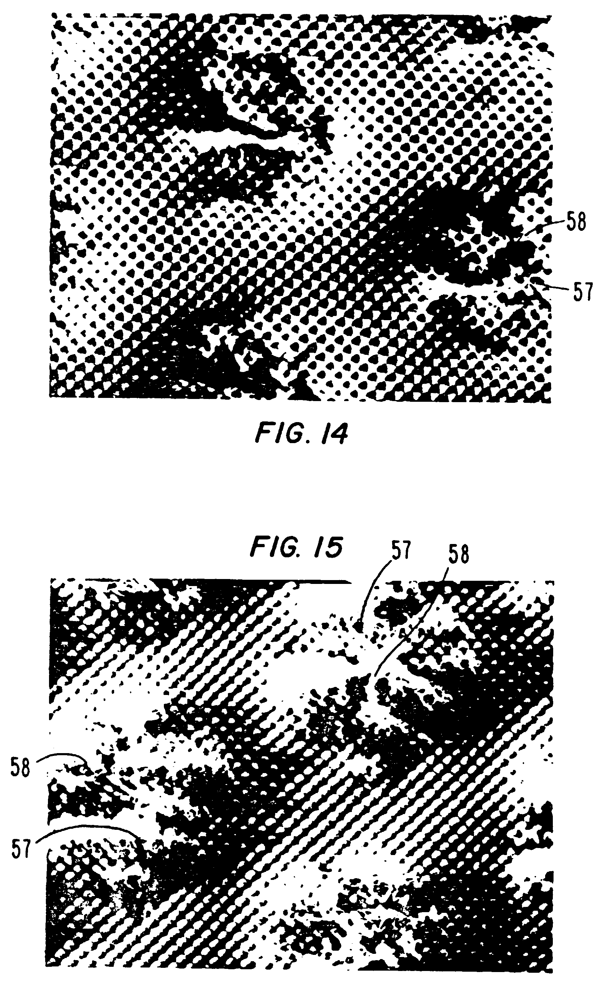

A third embodiment of an apertured plastic film in accordance with the present invention is illustrated in FIGS. 11-15 of the drawings. Apertured plastic film 50 comprises a thin layer of stretchable thermoplastic film comprising a plurality of micro-holes 57 which are provided or arranged in a plurality of groups or clusters 55 of micro-holes arranged in a regular pattern. Each cluster 55 comprises a plurality of rows 56 of micro-holes, five such rows being illustrated in FIG. 11. There are a large number of randomly distributed micro-holes 57 in each row 56, with these micro-holes being separated from one another in the rows by very fine, fiber-like elements 58. Adjacent rows 56 of micro-holes 57 are separated from one another in the clusters 55 by strips 59 of unapertured material. As illustrated, the regions 52 around and between neighboring clusters 57 are substantially free of micro-holes 57.

The thickness, T, of an apertured film of the invention is larger than the thickness, t, of the starting film from which it was derived. For example, the observed thickness, T, of the apertured film of FIGS. 1-5 is about 0.05 cm; and the thickness, T, of the apertured film of FIGS. 6-10 is about 0.064 cm.

The depth of valleys 33 is not uniform throughout the apertured film. Valleys 33 may vary somewhat in depth in going from one region of the apertured film to another. For example, the depth of a valley at the lower left-hand portion of FIG. 1 may be less than, the same as, or greater than the depth of that same valley at the upper right-hand portion of FIG. 1.

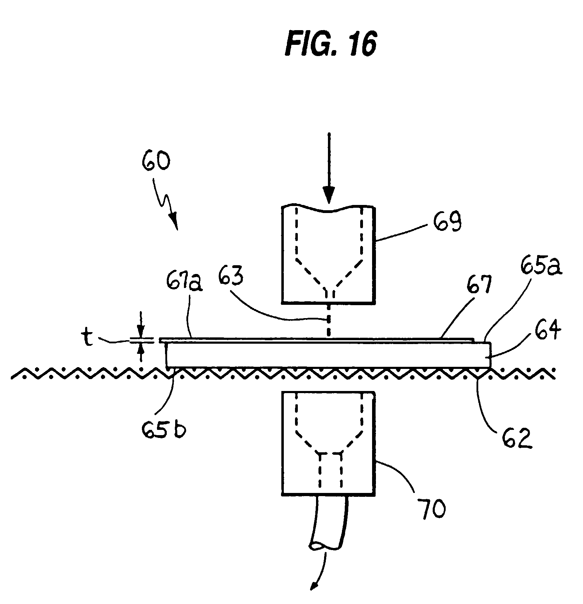

FIG. 16 is a schematic view showing an apparatus for making apertured plastic films of the present invention. Apparatus 60 comprises a movable conveyor belt 62 and, placed on top of this belt, to move with the belt, a backing member 64. The backing member has a plurality of openings (not shown in FIG. 16) disposed therein, said openings running through the thickness of the backing member from its upper surface 65a to its lower surface 65b.

Placed on top of the backing member is a thin, continuous, uninterrupted film 67 of thermoplastic polymeric material. This film may be vapor permeable or vapor impermeable; it may be embossed or unembossed; it may, if desired, be corona-discharge treated on one or both of its major surfaces or it may be free of such corona discharge treatment. The stretchable film may comprise any thermoplastic polymeric material including, by way of example, polyolefins, such as polyethylene (high, linear low or low density) and polypropylene; copolymers of olefins and vinyl monomers, such as copolymers of ethylene and vinyl acetate or vinyl chloride; polyamides; polyesters; polyvinyl alcohol and copolymers of olefins and acrylate monomers such as copolymers of ethylene and ethyl acrylate. Film comprising mixtures of two or more such polymeric materials may also be used. The machine direction (MD) and cross direction (CD) elongation of the starting film to be apertured should be at least 100% as determined according to ASTM Test No. D-882 as performed on a Instron test machine run at a jaw speed of 127 cm/minute (50 inches/minute). The thickness of the starting film (i.e. the film to be apertured) is preferably uniform and may range from about 0.0013 cm (0.0005 inch) to about 0.0127 cm (0.005 inch; preferably, the starting film has a thickness, t, between 0.0013 cm (0.0005 inch) and 0.0076 cm (0.003 inch). Co-extruded films can be used as can films which have been modified, e.g. by treatment with a surface active agent. The starting film can be made by any known technique such as casting, extrusion or blowing.

Situated above starting film 67 is a manifold 69 for applying a fluid 63, preferably water, to the upper surface 67a of the starting film as said film, supported on backing member 64, is moved with conveyor belt 62. The water may be applied at varying pressures. Disposed beneath the conveyor belt is a vacuum manifold 70 for removing water which is directed onto upper surface 67a of starting film 67 as it passes under manifold 69.

In operation, starting film 67 is placed on backing member 64 and the film and backing member are passed back and forth under manifold 69 a number of times until the desired apertured film is produced.

Manifold 69 comprises a plurality of holes which may range in number from about 11.8 per lineal cm (30 per lineal inch) to about 39.4 per lineal cm (100 per lineal inch). Preferably, the number of holes in the manifold ranges from about 13.8 per lineal cm (35 per lineal inch) to about 19.7 per lineal cm (50 per lineal inch). The holes are preferably circular in configuration and have diameters ranging from about 0.0076 cm (0.003 inch) to about 0.0254 cm (0.01 inch), preferably 0.0127 cm (0.005 inch) to 0.018 cm (0.007 inch). After the starting film and backing member are passed under manifold 69 a number of times, the application of the water is stopped and the application of vacuum is continued to assist in dewatering the resulting apertured film of the invention. The apertured film is removed from the backing member and dried by any convenient technique such as the application thereto of a warm air flow or by solvent extraction.

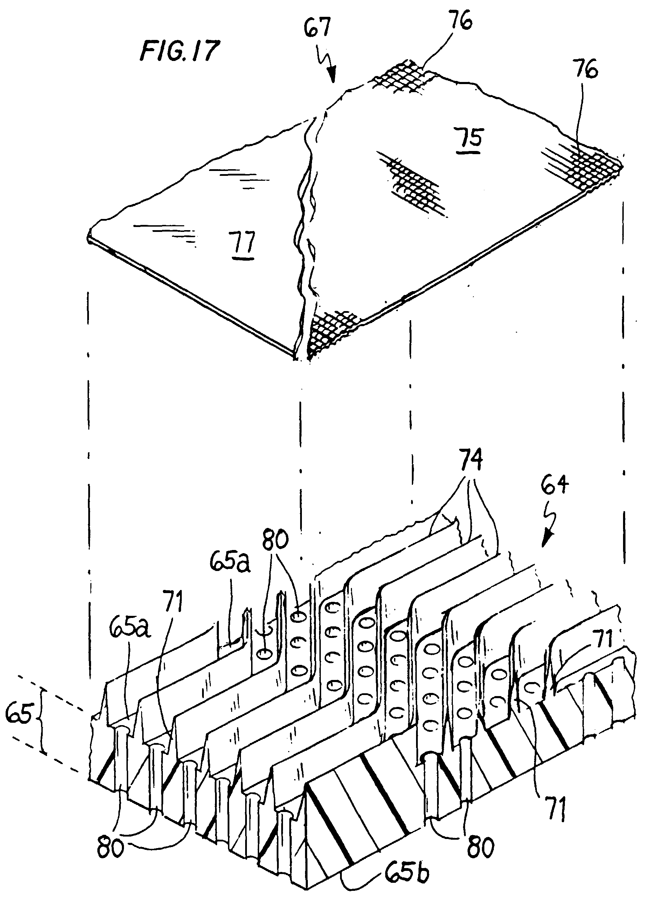

FIG. 17 is an exploded perspective view of certain parts, i.e. starting film 67 and backing member 64, described earlier herein in conjunction with FIG. 16. As mentioned earlier, starting film 67 comprises a thermoplastic polymeric material or mixture of two or more such polymeric materials and, as illustrated in FIG. 17, the film may be embossed or unembossed. A portion 75 of starting film 67 comprising embossments 76, and a portion 77 of unembossed film 67 are shown in the upper portion of FIG. 17.

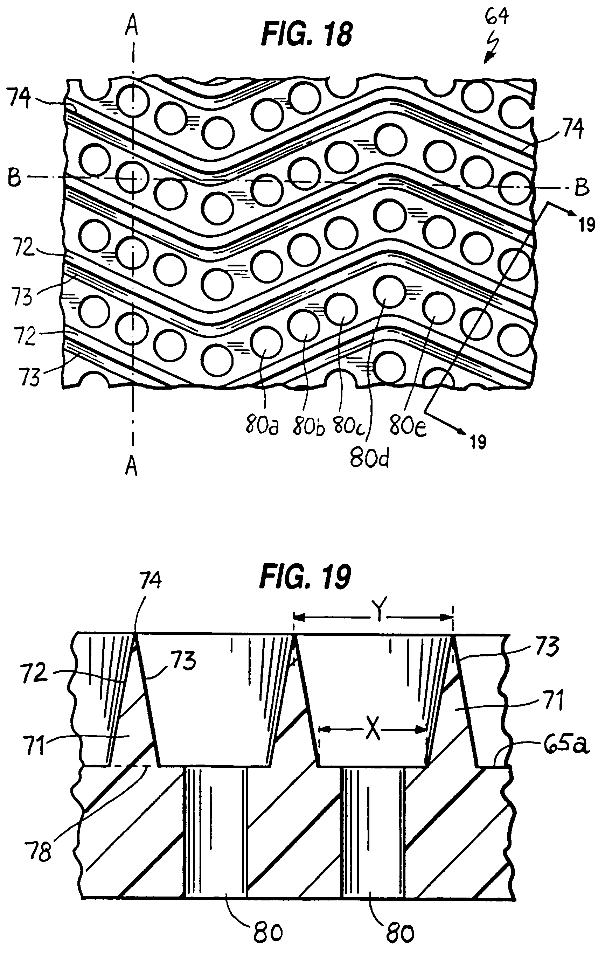

Backing member 64 comprises base portion 65 having an upper surface 65a and a lower surface 65b. Backing member 64 further comprises a plurality of apertures 80 running through the thickness of base 65 from upper surface 65a to lower surface 65b. As will be seen hereinafter, apertures 80 are provided to allow for removal of water during the manufacture of apertured film according to the invention. Backing member 64 also includes a plurality of vertically-extending support elements 71. These support elements comprise a base 78 coinciding with the plane of upper surface 65a of portion 65 and a pair of angled side walls 72,73 (best seen in FIGS. 18 and 19). Side walls 72,73 extend upwardly from base 78 to meet at a land portion or ridge 74. Support elements 71 are aligned in parallel and spaced equidistantly from one another. They may run either parallel to, perpendicular to, or at any angle to the sides of the backing member. As shown in FIGS. 17 and 18, support elements 71, when viewed in plan, are generally sinusoidal-like or wavy in configuration. It will be understood that the support elements may be provided in other configurations, e.g. straight-line, zig-zag and the like.

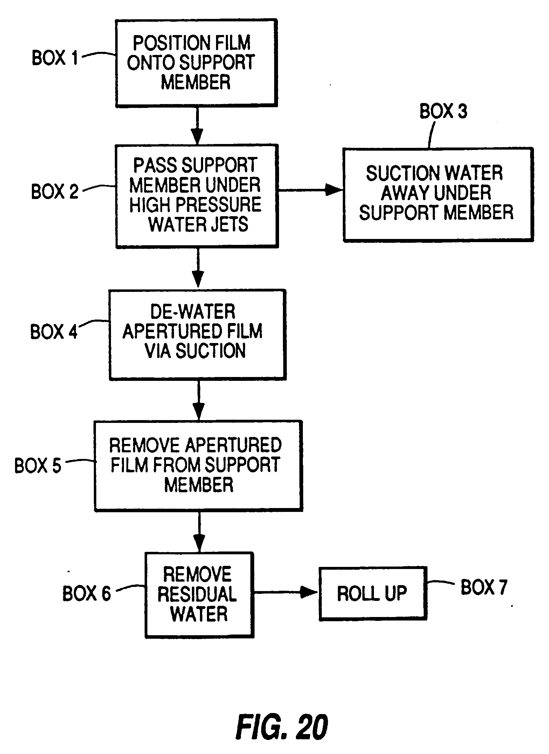

FIG. 20 is a block diagram showing the several steps in the process for producing the novel apertured films of the present invention. The first step in the process is to position a piece of thin, stretchable film of thermoplastic polymer material on a support member (Box 1). The support member with the stretchable film thereon is passed under high pressure fluid ejecting nozzles (Box 2). The preferred fluid is water. The water is transported away from the support member, preferably using a vacuum (Box 3). The film is de-watered, suction being preferred for this purpose (Box 4). The de-watered apertured film is removed from the support member (Box 5). Residual water is removed from the apertured film, e.g. by applying a stream of air thereto (Box 6). The apertured film is then rolled up to await use as is or as a structural component of another product such as a sanitary napkin, disposable diaper or wound dressing (Box 7).

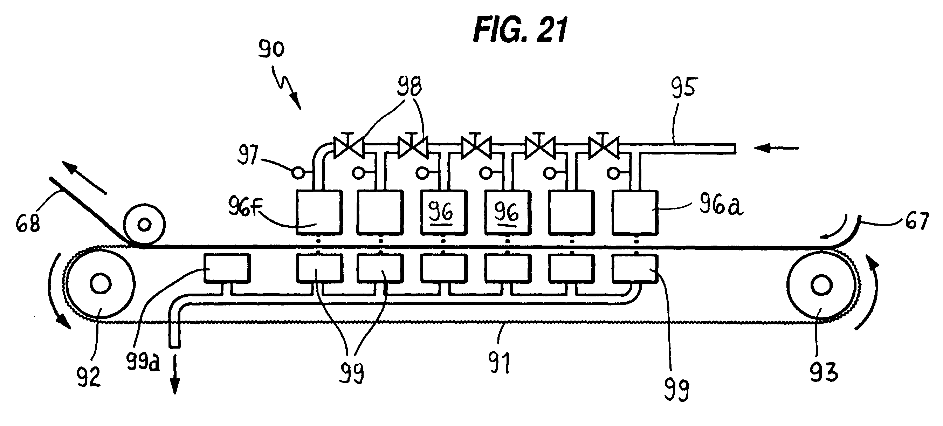

FIG. 21 is a diagrammatic view of an apparatus for continuously producing the apertured films of the present invention. Apparatus 90 comprises a backing member provided in the form of a conveyor belt 91. Conveyor belt 91 is continuously moved in a counterclockwise direction about a pair of spaced apart rollers 92,93 as is well-known. Disposed above conveyor belt 91 is a fluid supply manifold 95 connecting a plurality of lines or groups 96 of orifices. Each group 96 of orifices includes at least one row of very small diameter holes, there being 11.8 (thirty) or more of such holes per lineal cm (inch) in each row. Manifold 95 is equipped with pressure gauges 97 and control valves 98 for regulating the fluid pressure in each line or group of orifices. Means (not illustrated in the drawings) are provided for supplying water at an elevated temperature to manifold 95. Disposed beneath each orifice line or group is a suction member 99 for removing excess water during processing and to keep the aperturing zone from flooding. The starting film 67 to be formed into the apertured film 68 of the invention is fed to the conveyor belt comprising the backing member. The starting film passes under the group 96 of orifices where it is exposed to the columnar streams of water being ejected from the orifices. The pressure of the water columns being ejected from the individual groups 96 of orifices can be set by pressure control valves 98 to any desired pressure. The pressure of the water supplied to the groups 96 of orifices should be at least about 35.153 kg/cm$^2$ (500 psig) and may range up to 105.460 kg/cm$^2$ (1500 psig) or even higher. In the process for making apertured films of the present invention, it is preferred that the individual groups 96 of orifices eject water at the same pressure. Though six fluid supplying groups of orifices are shown in FIG. 21, the number of groups of orifices is not critical, but will depend on the thickness of the starting film, the speed of the conveyor belt, the pressures employed, the number of rows of orifices in each group of orifices 96, etc. After passing between the columnar water jets and suction manifold 99, the apertured film 68 passes over an additional suction slot 99a to remove excess processing water therefrom. The conveyor belt comprising the backing member may be made from relatively rigid material and may comprise a plurality of slats. Each slat extends across the width of the conveyor and has a lip on one side and a shoulder on the opposite side so that a shoulder of one slat engages the lip of an adjacent slat to allow for movement between adjacent slats and to allow for these relatively rigid slat members to be employed in the conveyor configuration shown in FIG. 21. Alternatively, the backing member may be a woven screen having high points which support the film and low points into which the film is moved during processing.

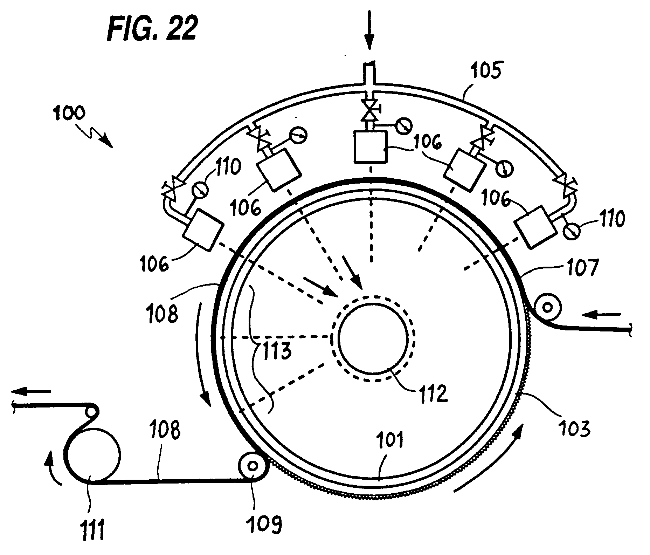

Referring to FIG. 22, there is shown a preferred apparatus for making apertured films of the present invention. Apparatus 100 comprises a rotatable drum 101. The drum has a honey-comb structure to allow for the passage of fluids therethrough, rotates in a counterclockwise direction and carries a backing member in the form of an elongated cylinder or sleeve 103 placed over its outer surface. Disposed about a portion of the periphery of the drum is a manifold 105 connecting a plurality of orifice strips 106 for applying water to a stretchable thermoplastic starting film 107 carried on the outer surface of sleeve 103. Each orifice strip comprises a row of very fine uniform circular holes. The diameter of these holes should range from approximately 0.0127 cm (0.005 inch) to 0.0254 cm (0.010 inch). There may be as many as 19.7 (50) or 23.6 (60) holes per lineal cm (inch) or more if desired. Water is directed under pressure through the orifices, forming columnar streams which impinge on the upper surface of the starting film in a contact or aperturing zone below the orifice strips. The pressure of the water supplied to the orifice strips is controlled by pressure control valves 109, the pressure being indicated by pressure gauges 110. The drum is connected to a sump 112 to which a vacuum may be applied to aid in removing water so as to keep the aperturing zone from flooding. In operation, the starting film 107 is placed on the backing member 103 before the water ejecting orifice strips 106. Film 107 passes underneath the orifice strips where it is formed into the apertured film of the invention.

## Example 1

The apertured plastic film shown in FIGS. 1-5 was made using apparatus 100 of FIG. 22 equipped with the backing member illustrated in FIGS. 17-19. The starting material was a 0.00254 cm (1.0 mil) thick embossed film comprising 50% by weight of linear low density polyethylene and 50% by weight low density polyethylene. The film was obtained from Exxon Corporation under the designation EMB-533. This film had a softening point of 80°C, a melting point of 120°C, a specific gravity of 0.91 and a % elongation to break of 460 in the machine direction and 630 in the cross-direction. The film has a MD tensile strength of 53.57 kg/m (3.0 lbs/in) and a CD tensile strength of 44.64 kg/m (2.5 lbs/in).

Backing member 103 was provided in the form of a cylindrical sleeve which was placed over support drum 101 as shown in FIG. 22. The outer surface of the backing member bad the configuration illustrated generally in FIGS. 17-19. Base 78 measured 0.076 cm (0.030 inch). The height of support elements 71, measured from base 78 to ridge 74, was 0.191 cm (0.075 inch). There were 4.7 (12) such support elements 71 per cm (inch) as measured along reference line A-A of FIG. 18. Support members 71 were aligned circumferentially on the outer surface of the sleeve, i.e. so that reference line B-B shown in FIG. 18 was parallel to the sides of the sleeve.

As shown in FIG. 19, the distance X between adjacent support members 71 at the upper surface 65a of base portion 65 was 0.152 cm (0.06 inch). The distance Y between adjacent support members at their ridges 74 was 0.229 cm (0.09 inch). Apertures 80 were circular and had a diameter of 0.091 cm (0.036 inch). Referring to FIG. 18, apertures 80a, 80b, and 80c were spaced at a center-to-center distance of 0.112 cm (0.044 inch), while aperture 80d was spaced at a center-to-center distance of 0.145 cm (0.057 inch) from adjacent apertures 80c and 80e. This aperture spacing pattern was uniformly employed throughout the backing member.

The cylindrical sleeve comprising backing member 103 was made from Celcon CE-4 acetal copolymer resin available from Hoechst-Celanese. Other materials, such as aluminum, which can be formed into the mentioned cylindrical sleeve may be employed if so desired.

In the process employed for making the apertured film of this Example 1, only three of the five groups 106 of orifices shown in FIG. 22 were used. Each group 106 of orifices bad a single row of orifices of diameter 0.0127 cm (0.005 inch), there being 19.7 (50) such orifices per lineal cm (inch). Each group 106 of orifices was located so that the exits of the orifices were 2.22 cm (0.875 inch) above the ridges 74 of vertical support elements 71. Heated water was supplied to water supply manifold 105 where it was distributed to the three groups 106 of orifices. The water was supplied to the three groups of orifices at a pressure of about 43.09 kgcm$^{-2}$ (613 psig) and at a temperature of 71.1°C (160°F). Starting film 107 was conducted from a let-off roll (not shown in FIG. 22) and led onto the outer surface of the backing member 103 having the sinusoidal-like ridge structure just described. It will be understood that the film was supported on ridges 74 during processing. Support drum 101 carrying the backing member 103 was rotated at 15.24 m/min (50 ft/min) by means of a drive mechanism (not illustrated in the drawings). Water passing through apertures 80 in the backing element 64 and through support drum 101 was removed under suction via sump 112 and was recirculated to the water-supply manifold 105. After passing under the last of the groups 106 of orifices, the now apertured film 108 was led over a de-watering zone 113. Apertured film 108 was then removed from the backing member by stripper roll 109 and passed over the outer surface of a perforated cylinder 111 operating under vacuum to remove any residual processing water prior to further handling or processing.

The apertured film made according to the process of this Example 1 had the structure described earlier herein in connection with FIG. 1-5 of the drawings.

## Example 2

Another apertured film was made using the apparatus and process described in Example 1 except the water was supplied to the three groups 106 of orifices at a pressure of about 42.18 kgcm$^{-2}$ (600 psig). The starting material was a 0.00254 cm (one mil) thick embossed film comprising a blend of low density and linear low density polyethylene. The film was supplied by Edison Plastic of Edison, New Jersey as MFST 141. This film has a machine direction tensile strength of 58.93 kg/m (3.3 lbs/in). and an elongation at break of 245 %; and a cross machine tensile strength of 39.29 kg/m (2.2 lbs/in). and an elongation at break of 650%. The resulting apertured film was tested and found to have the following properties:

| | |
|---|---|
| Machine direction (MD) tensile strength: | 0.86 kg (1.9 lbs) |
| Cross direction (CD) tensile strength: | 0.54 kg (1.2 lbs) |
| % Open Area | 3.0 |
| Mean equivalent hydraulic diameter, cm (mils), of micro-holes | 0.0076 (3.03) |
| Coefficient of Variation (COV) of micro-hole size | 162% |
| Bulk, cm (mils) | 0.0584 (23) |
| Strike-through time, seconds | 27 |
| MD tensile strengths and CD tensile strengths were determined on 2.54 cm (1") wide samples of apertured film using an Instron Testing Machine in accordance with ASTM Test Method D-882. <br> % open area was determined on a Quantimet Q520 Image Analyzer sold by Cambridge Instruments Ltd. | |

The open area and EHD of the apertured film were determined by image analysis. Essentially image analysis converts an optical image from a light microscope into an electronic signal suitable for processing. An electronic beam scans the image, line by line. As each line is scanned an output signal changes according to illumination. White areas produce a high voltage and black areas a low voltage. An image of the apertured film is produced and, in that image, the holes in the apertured film are white while the solid areas of thermoplastic material are at various levels of grey. The more dense the solid area, the darker the grey area produced. Each line of the image that is measured is divided into sampling points or pixels. Such analysis is carried out by using a Quantimet Q520 Image Analyzer sold by LEICA/Cambridge Instruments Ltd. The analyzer uses Version 4.0 software with Grey Store Option. The light microscope used is an Olympus SZH Microscope with transmitted light base and plan 0.5X objective. The image is produced with a COHU video camera.

Apertured films are prepared for analysis by sputter coating (30 seconds) in a Polaron II HD sputter coater to render transparent film areas opaque. Samples are mounted on 7.62 cm (3 inch) x 10.16 cm (4 inch) glass slides using double faced tape (at the ends of the slides) to secure apertured films in position.

A representative piece of each apertured film to be analyzed is placed on the microscope stage and sharply imaged on the video screen at a magnification of 10x. The open area is determined from field measurements of representative areas. The Quantimet program output reports mean value and standard deviation for each sample.

The EHD (equivalent hydraulic diameter) of apertures are measured at a magnification of 20x. The Quantimet calculates EHD from the measured area and perimeter of each aperture according to the formula

$$EHD = 4\,\frac{A}{P}$$

where A = aperture area and P = aperture perimeter.

The Quantimet program output reports the EHD values and standard deviation for each sample.

EHD values can also be determined by the test procedure disclosed in U.S. 4,324,276 mentioned earlier herein.

Mean value, Standard Deviation (SD) and Coefficient of Variation (COV) are determined using standard statistical methods. COV is determined as follows:

$$COV = \frac{SD}{Mean} \times 100\%$$

Coefficient of Variation of EHD is an indication of the variability of the size of the micro-holes in the apertured film in terms of their Equivalent Hydraulic Diameter. If all micro-holes were of the same EHD, there would be no variation in EHD and the COV of EHD would be 0%. Larger COV's of EHD indicate larger variations in the sizes of the micro-holes in terms of their EHD. As the COV of EHD of apertured films made in accordance with the teachings of the present invention increases, the films increasingly take on the appearance of prior art nonwoven fabric derived from fibrous webs.

Liquid Strike-Through Values were determined as follows. The strike-through test measures the time required for 5cc of a test liquid to flow through an elliptical opening having a major axis of 3.81 cm (1.5 inches) and a minor axis of 1.9 cm (0.75 inch). The test fluid is a synthetic menstrual fluid designed to emulate the flow characteristics, viscosity, color, and ionic properties of human menstrual fluid. A 1.27 cm (0.5 inch) plexiglass plate with the described elliptical opening was placed on an absorbent pad of the type used in sanitary napkins, said absorbent pad being wrapped in the material to be tested. Five (5) cc of the aforementioned test liquid were then poured through the elliptical opening in the plexiglass plate. The strike-through time in seconds was recorded as the time elapsed between introduction and disappearance of the fluid on the surface of the material being tested.

## Example 3

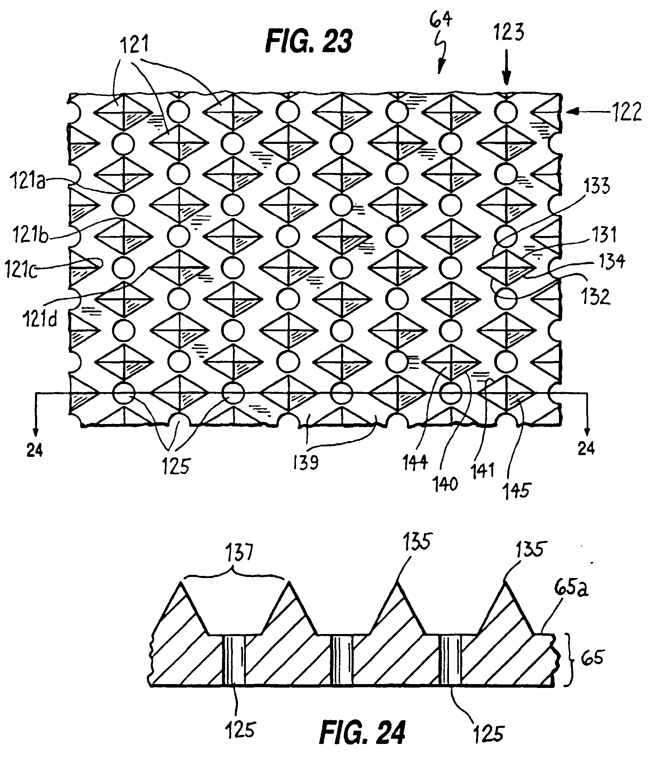

The apertured plastic film shown in FIGS. 6-10 was made using apparatus 100 of FIG. 22 equipped with backing member 103 shown in FIGS. 23 and 24 of the drawings. In this instance, the backing member 103 comprised a plurality of four-sided pyramids 121 arranged in rows 122 and columns 123. The pyramids extended generally vertically from the upper surface 65a of base portion 65 of backing member 64. The rows of pyramids were disposed horizontally and the columns of pyramids were disposed vertically as viewed in FIG. 23. The rows 122 of pyramids were aligned parallel to the axis of rotation of drum 101. The columns of pyramids were aligned perpendicular to the rows and in a direction parallel to the direction of rotation of drum 101, so that during operation of apparatus 100 the columns of pyramids were aligned in parallel with the machine direction of the apparatus and the machine direction of the thermoplastic film being processed thereon. As can be seen in FIG. 23, the pyramids in any given row were staggered with respect to the pyramids in each of the rows immediately adjacent thereto.

The backing element further comprised a plurality of apertures 125 running through the thickness of base portion 65. Apertures 125 were circular and had a diameter of 0.081 cm (0.032 inch). As seen in FIG. 23, apertures 125 were nearly, but not quite, in contact with each immediately adjacent pyramid 121 in a column 123; in other words, the diameter of apertures 125 was nearly equivalent to the spacing between the apices 121a, 121b of any two adjacent pyramids in a column 123. However, as can be seen in both FIGS. 23 and 24, apertures 125 were spaced a certain distance from each of the immediately adjacent pyramids 121 in a row 122; in other words the diameter of apertures 125 was less than the distance between the apices 121c, 121d of any two adjacent pyramids in a row 122.

The four side edges 131, 132, 133, 134 at the base of pyramids 121 were each 0.094 cm (0.037 inch) in length.

As seen in FIG. 24, the spacing 137 between peaks 135 of two immediately adjacent pyramids in a row 122 was 0.183 cm (0.072 inch). The spacing between peaks 135 of two immediately adjacent pyramids in a column 123 was .105 cm (0.0415 inch). Pyramids 121 defined a plurality of intersecting valleys 139 in the backing element, all such valleys being 0.089 cm (0.035 inch) in width as measured perpendicularly from edge 140 of pyramid 144 to edge 141 of adjacent pyramid 145.

The starting film, i.e. Exxon EMB-533, using in Example 1 was also used to make the apertured film of this Example 3. Apparatus 100 (except for the backing element) used for this Example 3 was identical to that used in Example 1. Water was supplied to the three groups of orifices at a pressure of about 112.5 kgcm$^{-2}$ (1600 psig) and at a temperature of 32.2°C (90°F). Support drum 101 carrying the backing member described with reference to FIGS. 23 and 24 was rotated at 15.24 m/min. (50 ft/min).

The resulting apertured film had the structure described earlier herein with reference to FIGS. 6-10. The film had the following physical properties:

| MD tensile strength, kg/m (lbs./inch) | 19.6 (1.1) |
|---|---|
| CD tensile strength, kg/m (lbs./inch) | 23.2 (1.3) |
| % open area | 7.02 |
| Bulk, cm (mils) | 0.068 (27) |
| Strike-through time, secs. | 28 |

Referring to FIG. 6, the mean EHD of micro-holes 47 in the apertured film of this Example 3 was 0.00858 cm (3.38 mils). The Coefficient of Variation of the EHD of micro-holes 47 was 157%. The apertured film also included a plurality of larger holes 42, these larger holes 42 having been produced when the columnar water jets forced the starting film over the pointed upper surfaces of pyramids 121 of the backing during processing. The mean EHD of larger holes 42 in the apertured film of this Example 3 was 0.044 cm (17.32 mils). The wide variation in micro-hole size, as indicated by the COV of EHD of 157%, provided an apertured film whose visual appearance was much more nearly like that of a nonwoven fabric made from a fibrous web. It will be recognized that larger holes 42 in the apertured film under discussion can be eliminated by providing the pyramids with apices which are rounded off, rather than pointed.

The apertured film of Example 3 was found to be particularly useful as facing layer for sanitary napkins.

### Example 4

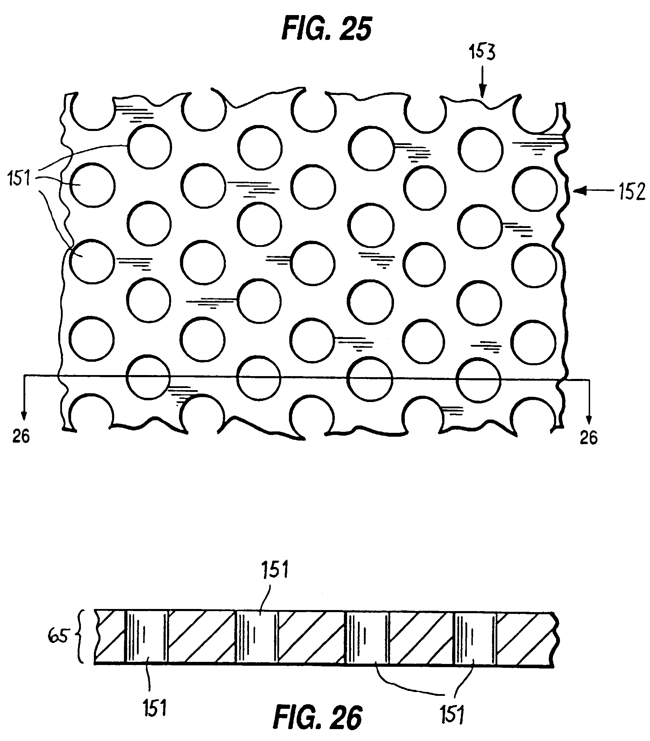

The apertured plastic film shown in FIGS. 11-15 of the drawings was made on apparatus 100 of FIG. 22 equipped with cylindrical backing member 103 having the configuration shown in FIGS. 25 and 26. Backing member 103 comprised a base portion 65 with a plurality of circular openings 151 arranged in rows 152 and columns 153. Rows 152 were disposed horizontally and columns 153 were disposed vertically as viewed in FIG. 25. Rows 152 were aligned parallel to the axis of rotation of support drum 103. Columns 153 were aligned perpendicularly to rows 152 and parallel to the direction of rotation of drum 101, so that during processing on apparatus 100, columns 153 ran parallel to the machine direction (MD) of the apparatus and the machine direction of the film undergoing processing. As can be seen in FIG. 25, the circular openings in any given row were staggered or offset in the horizontal direction with respect to the circular openings in each of the rows immediately adjacent thereto.

Circular openings 151 had a diameter of about .103 cm (0.041 inch). The center-to-center distance between the circular openings in the rows 152 was about 0.17 cm (0.067 inch), while the center-to-center distance between the circular openings in the columns 153 was about .104 cm (0.041 inch).

Backing member 103 having the configuration shown in and just described with reference to FIGS. 25 and 26 was provided in the form of a cylindrical sleeve which was placed over support drum 101. The starting film was Exxon EMB-533 used in preceding Examples 1-3. The temperature of the water supplied to the four groups 106 of orifices was 21.1°C (70°F). The water was supplied to the groups of orifices at a pressure of about 56.2 kg/cm$^2$ (800 psig). The water was ejected from the orifices in the form of very fine columnar streams. Support drum 101 was rotated at a speed of about 1.5 m/min (5 ft/min). The resulting apertured film has the structure shown in and hereinbefore described with reference to FIGS. 11-16 of the drawings.

The apertured films of Examples 2 and 3 were tested for % open area; Equivalent Hydraulic Diameter (EHD); Coefficient of Variation (COV) of EHD; Range of Hole Size; Coefficient of Variation of Hole Size; Shape Factor; Coefficient of Variation of Shape Factor; and width of the fiber-like elements. % open area, EHD, COF of EHD and size of holes were determined in accordance with the methods described earlier herein.

Shape Factor is an indication of the roundness of a hole or opening in a material being tested. Shape Factor (SF) is defined by the formula:

$$SF = \frac{P^2}{KA}$$

where P is the perimeter of the hole or opening being measured, A is the area of the hole or opening and K is a constant equal to 4Π.

For a hole or opening which is perfectly circular in configuration, the Shape Factor, SF, is unity, i.e. 1. The higher the value of the Shape Factor, the more irregular, i.e. the less circular, is the configuration of the hole or opening.

Materials having high Coefficients of Variation (COV) of hole size are much more textile-like in appearance to the viewer, i.e. the materials appear to be much more similar visually to traditional nonwoven fabrics made from fibrous webs and much less visually similar to plastic films having substantially uniform holes or openings.

Prior art facing materials were used as controls in the tests being discussed. The first control facing material, Sample C in Table I, was the facing material used on and taken from Kotex brand Super-Maxi sanitary napkins sold commercially by Kimberly-Clark Corporation. The second control facing material, Sample D in Table I, was the facing material used on and taken from Always brand Maxi-Pads sanitary napkins sold commercially by The Procter & Gamble Company. A third control facing material, designated Sample E in Table I, was the facing material used on Sure & Natural brand sanitary napkins sold commercially by Personal Products Company. In Table I, Sample A corresponds to the apertured film made in accordance with Example 2 hereof and Sample B corresponds to the apertured film made in accordance with Example 3 hereof.

The facing material designed Sample C in Table I is a liquid-permeable nonwoven fabric comprising continuous length fibers having substantially uniform diameters of about 0.003 cm (1.2 mils), this material being known in the art as a spun-bonded nonwoven fabric.

The facing material designated D in Table I is an apertured plastic material described on the outer package containing the sanitary napkins from which it was taken as "Dri-Weave Covering". This material is evidently made by the process disclosed in U.S. Patents 4,342,314 and 4,463,045 which are listed on said outer packaging.

The facing material designated sample E in Table I was made according to the teaching of U.S. 4,690,679.

TABLE I

| Property | SAMPLE | | | | |
|---|---|---|---|---|---|
| | A | B | C | D | E |
| % Open Area | 3.0 | 10.0 | 17 | 26 | 28 |
| COV of Shape Factor, % | 32 | 29 | 30 | 11 | 17.9 |
| EHD, openings | 95% < 25 mils < 0.0635 cm | 90% < 25 mils < 0.0635 cm | 95% < 25 mils < 0.0635 cm | 75% > 25 mils > 0.0635 cm | 95% between 0.0254-0.0457 cm (10-18 mils) |
| COV of EHD. % | 80 | 87 | 92 | 11 | 7 |
| Hole Size, $cm^2$ x $10^{-6}$ (square mils) | 95% between 0.0466 (0.3) and 62 (400) | 95% between 0.0466 (0.3) and 62 (400) | 95% between 0.0466 (0.3) and 62 (400) | 80% between 62 (400) and 116.6 (750) | 95% between 13.99 (90) and 62 (400) |
| COV of Hole Size, % | 160 | 190 | 200 | 11 | 24.5 |
| Width of fiber-like elements, cm (mils) | 80% between 0.00254 (1) and 0.0127 (5) | 80% between 0.00254 (1) and 0.0127 (5) | 100% 0.00305 (1.2)* | 100% between 0.0381 (15) and 0.0762 (30)# | 100% between 0.0381 (15) and 0.0889 (35)# |

* = fiber diameters
# = width of plastic material between holes

The test results are shown in Table I. It can be seen from these test results that the apertured thermoplastic films of the present invention (Table I, Samples A and B) are very similar to nonwoven fabrics made from fibrous webs (as exemplified by the spunbonded nonwoven fabric Sample C) and are quite dissimilar from the prior art apertured plastic materials exemplified by Samples D and E.

Sanitary napkins were made utilizing the liquid permeable apertured films of Examples 2 hereof and the facing materials described earlier herein and designated C and E in Table I. The napkins were tested for strike-through times, rewet properties and color masking properties.

The sanitary napkins which were tested contained absorbent cores of the type used commercially in Sure & Natural sanitary napkins sold by Personal Products Company and were wrapped with facing materials A, C and D. The facing materials were overlapped and sealed on the lower surface of the test napkins as known in the art, leaving a single layer of facing material on the upper, i.e. body contacting, surface of the napkins.

Strike-through time in seconds for the test sanitary napkins were determined according to the method

EP 0 626 838 B1

described earlier herein. After completion of the liquid strike-through test, two plies of 7.62 cm (3") x 10.16 cm (4") filter paper were placed over the area of the sanitary napkin stained by the synthetic menstrual fluid applied during the liquid strike-through test procedure. A 1.81 kg (four (4) pound) roller, 12.7 cm (5 inches) in length and 8.26 cm (3.25 inches) in diameter, was rolled back and forth once over the entire upper surface of the napkin being tested. The amount in grams of synthetic menstrual fluid absorbed by the filter papers was taken as the "rewet value".

Stain-masking properties of various cover materials were determined with the use of a Hunter Color Meter using the following procedure. Sanitary napkins for testing were prepared by wrapping absorbent cores of wood pulp fluff with the cover materials to be tested as described earlier herein. The sanitary napkins to be tested were placed over the specimen part of the Hunter Color Meter and covered with a black calibration glass. Readings were then taken on the L, a and b scales of the meter. This procedure was repeated two more times on different parts of the napkin being tested. The opacity value, OV, of the sample under test was determined by the formula:

$$OV = \frac{M}{N}\,(100)$$

where M = the digital read-out value on the Hunter Color Meter with the black calibration glass in place; and

where N = the digital read-out value on the Hunter Color Meter with the white calibration glass in place.

The sanitary napkins to be tested are covered with the plexiglass plate used to determine strike-through times. Five (5) cc of the menstrual fluid described earlier herein were poured through the elliptical opening in the plate and allowed to be absorbed by the napkin, simulating the stain which would result from contact by actual menstrual fluid. The intensity of the stain in the stained region of the sanitary napkin was then measured by taking reading on the L, a and b scales of the Hunter Color Meter. Stain Intensity, SI, was determined by the formula:

$$Stain\ Intensity = [(L_s - L_{us})^2 + (A_s - A_{us})^2 + (b_s - b_{us})^2]^{\frac{1}{2}}$$

where subscript s = value obtained for stained region and us = value obtained on the unstained control.

Stain Masking Values, SMV, for each covered sanitary napkin was then determined by the formula:

$$SMV = OV - SI$$

where OV is the opacity value and SI is the Stain Intensity.

The test results are reported in Table II.

TABLE 2

| Property | COVER MATERIAL USED ON SANITARY NAPKIN | | |
|---|---|---|---|
| | A | C | D |
| Strike-through time, secs. | 27 | 16 | 16 |
| Rewet value, grams | 0.18 | 0.53 | 0.46 |
| Stain Masking | 70 | 58 | 64 |
| Open Area, % | 3 | 17 | 26 |
| Note: Cover material C is a nonwoven fabric which has tortuous paths for passage of fluids. The open area determined by transmitted light measurements may not reflect the total area which allows fluids to pass. | | | |

Cover materials A, C and D in Table II correspond to cover materials A, C and D in Table I.

The test results reported in Table II demonstrate that the apertured thermoplastic films of the present invention, as exemplified by the apertured film made according to Example 2 (cover material A, Table II) provide sanitary napkins whose stain-masking properties and rewet values are equal to or better than those of commercially available sanitary napkins utilizing either a spun-bonded nonwoven fabric (i.e. cover material C, Table II) or a plastic apertured cover material (i.e. cover material D, Table II), and whose strike-through times, though somewhat higher than those of existing commercially available products, are nevertheless functionally acceptable. These favorable results are quite surprising in light of the fact that the apertured plastic film of Example 2 hereof significantly lower has an open area than the prior art cover materials C and D.

A Fourier Transform Infra-Red (FTIR) instrument with a microscope attachment was employed to identify draw ratio differences between the fiber-like elements and the polymeric film material portions adjacent those fiber-like elements in the apertured film products of the present invention. It is to be appreciated that in processing the material of the present invention, the starting film is supported on the high points of the backing element and under the influence of the impact of the columnar streams of water the unsupported portions of the starting material are caused to deflect toward and partially into the depressed regions or valleys of the backing element.

This deflection or stretching continues until the starting film breaks into the micro-holes and fiber-like elements characteristic of the apertured films of the invention. The stretching or drawing is localized in that most of the stretching or drawing occurs in those portions of the starting film which become the fiber-like elements. The purpose of this test is to determine the difference in the stretch which has occurred in the fiber-like elements and in the polymeric material adjacent the areas of fiber-like elements.

These determinations were made using a dual bench FTIR spectrometer (Model 1800 FTIR, Perkin-Elmer Corp., Norwalk, CT) equipped with a research grade IR microscope (IRPLAN infrared microscope, Spectra Tech, Stamford, CT) operated in the transmittance mode. IR spectra were obtained using a medium band, 1 mm$^2$, mercury-cadmium-telluride (MCT) detector, and a 15X cassigrain (IR) objective lens. The spectra were scanned at a resolution of 2 cm$^{-1}$ between 4000 and 600 cm$^{-1}$. The data interval used was limited to 1 (sec./spectrum) for a resolution of 2 cm$^{-1}$. The operational mode parameter for the background and the apertured film samples examined were the same except for the Jacquinot stop which was 1 for the background and 4 for the processed samples. The normal Hays-Ganzel apodization function was used in all cases. All specimens were scanned 200 times with the necessary apertures in place. One collection of background scans were used to ratio the subsequent test sample spectra. The background spectrum was acquired using a 1 mm aperture in the condenser, a 1 $\mu$m pinhold in the sample tray, and a 1.5 mm aperture in the upper optical module. The sample spectra was acquired using redundant aperturing above and below the sample so as to pinpoint the specific area of interest in the samples to be examined.

A draw ratio calibration curve was developed by mechanically deforming the starting polymeric film material measured amounts of elongation from 0 to 500% in 100% increments and obtaining FTIR spectra for each sample.

Spectra were then obtained from fiber-like elements in the apertured film material and from the areas immediately adjacent those fiber-like elements. These spectra were compared to the spectra of the calibration curve and the degree of elongation of the fiber-like elements was determined therefrom. It was found that the fiber-like elements of the apertured film of the invention had been drawn at least 100% as compared to adjacent regions of starting film which had not undergone aperturing during processing. A major portion of the fiber-like elements were found to have been drawn from about 200% to about 250% compared to adjacent regions of starting film which had not undergone aperturing during processing.

As indicated earlier herein, apertured films of the present invention may be used as facing materials for absorbent products such as disposable diapers, sanitary napkins, wound dressings, incontinent devices and the like.

When used as covering materials for sanitary napkins, it is preferred that the micro-holes of the apertured thermoplastic films of the present invention be sufficient in number to provide an open area ranging from about 1-15%, with the number of larger-sized holes (such as those numbered 42 in FIG. 6 of the drawings) preferably being minimized. It is preferred that at least fifty percent (50%) of the micro-holes comprising the film have EHD's ranging between 0.00127 and 0.0635 cm (0.5 and 25 mils). The COV of EHD of the micro-holes is preferably at least 50%. Preferably, at least seventy-five percent (75%) of the micro-holes have areas less than 62 x 10$^{-6}$ cm$^2$ (400 square mils) and the Coefficient of Variation of micro-hole area should be at least 100%.

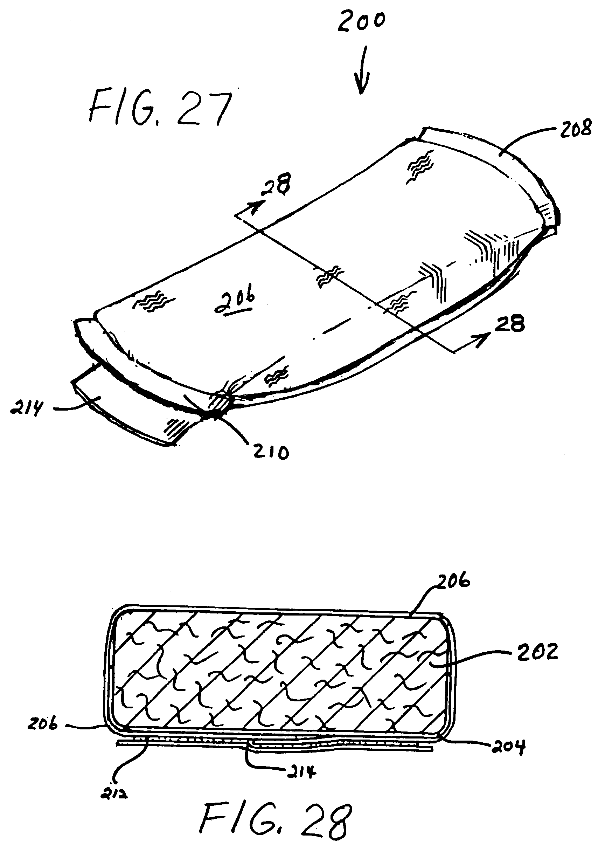

Referring to FIGS. 27 and 28, there is shown a sanitary napkin 200 comprising an absorbent core 202 of wood pulp fibers, a thin, fluid-impermeable barrier film 204 and a covering material 206 which may be any of the apertured films of the invention. Preferably, the covering material has the structure shown and described herein with reference to FIGS. 1-5 and Example 2. Barrier film 204, which may comprise, e.g. a thin film of polyethylene, contacts the lower surface of absorbent core 202 and runs part way up the longitudinal sides of the absorbent core. Covering material 206 has a length somewhat longer than the length of the absorbent core and is wrapped around the absorbent core and barrier film as shown in FIG. 28. The longitudinal edges of the cover material are overlapped and sealed together on the lower surface of the napkin in the usual manner. In the embodiment illustrated, the cover material is sealed to itself at the ends 208,210 of the sanitary napkin. As illustrated in FIG. 28, sanitary napkin 200 has a layer of adhesive 212 for adhering the napkin to the undergarment of the user. Adhesive 212 is protected prior to use by a removable release strip 214.

Variations can be made in the structure of the backing element described herein. For example, the pyramids shown in FIG. 23 may have 3, 5 or more sides. The backing element could also have upstanding support elements in the form of cones, square bosses, etc.

## Claims

1. An apertured film comprising a polymeric material having a plurality of micro-holes therein, characterised in that the polymeric material comprises a stretchable thermoplastic polymeric material and the micro-holes (37) are defined by a network of fiber-like elements (38), wherein the thermoplastic polymeric material comprising said fiber-like elements (38) has been drawn, the said fiber-like elements having been drawn at least 100 percent as compared to adjacent regions of starting film which had not undergone aperturing during processing.

2. An apertured film (30) according to claim 1 wherein said micro-holes (37) have an area ranging from $0.019 \times 10^{-6}$ sq cm (0.003 square mils) to $2580 \times 10^{-6}$ sq cm (400 square mils).

3. An apertured film (30) according to claim 1 or claim 2 wherein the micro-holes (37) have a coefficient of variation of shape factor of at least about 25%.

4. An apertured film (30) according to any one of claims 1 to 3 wherein the micro-holes (37) have an equivalent hydraulic diameter ranging from $1.27 \times 10^{-3}$ cm (0.5 mil) to $63.5 \times 10^{-3}$ cm (25 mils).

5. An apertured film (30) according to any one of claims 1 to 4 wherein the micro-holes (37) have a coefficient of variation of EHD of at least about 50%.

6. An apertured film (30) according to any one of claims 1 to 5 having an open area of from 1% to 15%, said open area being provided by said micro-holes (37).

7. An apertured film (30) according to any one of claims 1 to 6 wherein the fiber-like elements (38) have lengths ranging from 0.013cm (0.005 inch) to 0.127cm (0.05 inch).

8. An apertured film (30) according to any one of claims 1 to 7 wherein the fiber-like elements (38) have widths ranging from 0.003cm (0.001 inch) to 0.089cm (0.035 inch).

9. An apertured film (30) according to any one of claims 1 to 8 wherein the fiber-like elements (38) have thicknesses ranging from 0. 0006cm (0.00025 inch) to 0. 005cm (0.002 inch).

10. An apertured film (30) according to any one of claims 1 to 9 wherein said micro-holes (38) are provided in a pattern of distinct groups.

11. An apertured film (30) according to any one of claims 1 to 9 wherein said micro-holes (38) are irregular in shape and are randomly distributed in the apertured regions of the film.

12. An apertured film (30) according to any one of claims 1 to 11 further comprising a plurality of secondary openings (42) whose areas are substantially larger than the areas of said micro-holes (38).

13. An apertured film (30) according to any one of claims 1 to 12 comprising a series of parallel ridges (32) and a series of valleys (33) between said series of ridges, each of said ridges (32) being formed by a pair of generally vertically oriented side walls (31) which meet on the upper surface of said film, said valleys comprising the plurality of micro-holes (37) defined by the network of fiber-like elements (38).

14. An apertured film (30) according to claim 13 wherein the lower reaches of said angled side walls (31) include a plurality of said micro-holes (37).

15. An apertured film (30) according to claim 13 or claim 14 wherein said ridges (32), when viewed in plan, have a sinusoidal-like configuration.

16. An apertured film (40) according to any one of claims 1 to 12 comprising a plurality of generally vertically oriented cone-like structures (41) defined by sloping side walls (41a), said sloping side walls defining a plurality of valleys (43) between said cone-like structures (41), said valleys (43) comprising the plurality of micro-holes (47) defined by the network of fiber-like elements (48).

17. An apertured film (40) according to claim 16 wherein the lower reaches of said sloping side walls (41a) include a plurality of said micro-holes (47).

18. An apertured film according to claim 16 or claim 17 wherein said cone-like structures (41) include secondary openings whose areas are substantially larger than the area of said micro-holes (47).

19. An apertured film (50) according to any one of claims 1 to 9 comprising said micro-holes (57) being arranged in a plurality of clusters (55) of micro-holes.

20. An apertured film (50) according to claim 19 wherein each said cluster (55) comprises at least one row (56) of said micro-holes (57), said row (56) of micro-holes (57) having a strip of unapertured material on either side thereof.

21. An apertured film (50) according to claim 19 or claim 20 wherein each said cluster (55) comprises a plurality of rows (56) of said micro-holes (57) and adjacent rows (56) are separated from one another by strips (59) of unapertured material.

22. An apertured film (50) according to any one of claims 19 to 21 wherein the regions (52) around and between neighbouring clusters (55) of micro-holes (57) are substantially free of micro-holes.

23. An absorbent product comprising an absorbent core covered with an apertured film, said apertured film being according to any one of claims 1 to 22.

24. An absorbent product according to claim 23 which is a wound dressing.

25. An absorbent product according to claim 23 or claim 24 wherein both major surfaces to said absorbent core are covered with said apertured film.

26. An absorbent product according to claim 23 which is a disposable diaper.

27. An absorbent product according to claim 23 which is a sanitary napkin.

28. An absorbent product according to claim 23 further comprising a liquid impervious backing layer located adjacent one major surface of said absorbent core.

29. A sanitary napkin comprising an absorbent core having an upper major surface and a lower major surface; a liquid-impermeable barrier layer; and a cover material, said covering material being in contact with at least the upper surface of said absorbent core, said covering material comprising an apertured film according to any one of claims 1 to 22, and said barrier layer being disposed adjacent the lower surface of said absorbent core.

30. A sanitary napkin according to claim 29 wherein at least 75% of the micro-holes have areas less than 400 square mils.

31. A method for forming an apertured film comprising a polymeric material having a plurality of micro-holes therein, said method comprising:

   a) providing a starting film (67) comprising a polymeric material and having an upper surface and a lower surface;

   b) providing a backing member (64) comprising localized support regions (74) for supporting a film (67), recessed zones into which the film may be deformed by the application thereto of fluid forces; and means (80) for allowing said applied fluid to be transported away from said backing member;

   c) supporting said starting film (67) on said backing member (64) with the lower surface of said film being in contact with the support surface of said backing member and with the upper surface of said film facing away from said backing member;

   d) directing a fluid force against the upper surface of said starting film (67) in a zone of contact and at a pressure sufficient to cause the formation of a plurality of said micro-holes;

e) moving said film (67) from said contact zone; and

f) removing said now-apertured film (68) from said backing member,

characterised by:

(a) providing a starting film (67) comprising a stretchable thermoplastic polymeric material; and

(d) directing a fluid force in the form of columnar streams to cause the formation of a plurality of micro-holes and to draw the thermoplastic polymeric material at least 100 per cent as compared to adjacent regions of starting film which had not undergone aperturing during processing so as to provide a network of drawn fiber-like elements defining said micro-holes.

32. A method according to claim 31 wherein said fluid is under pressure ranging from 351550 $kgm^{-2}$ (500 psig) to 1124960 $kgm^{-2}$ (1600 psig).

33. A method according to claim 31 or claim 32 wherein said fluid is water which is directed at the upper surface of said film at a temperature of at least about 32.2°C (90°F).

**Patentansprüche**

1. Gelochte Folie aus einem Polymermaterial mit einer Vielzahl von Mikrolöchern, dadurch gekennzeichnet, daß das Polymermaterial ein dehnbares thermoplastisches Polymermaterial ist und die Mikrolöcher (37) durch ein Netzwerk faserartiger Elemente (38) begrenzt sind, wobei das thermoplastische Polymermaterial aus den faserartigen Elementen (38) gestreckt worden ist und die faserartigen Elemente (38) im Vergleich zu angrenzenden Bereichen der Startfolie, die während der Bearbeitung nicht der Lochung unterlagen, um mindestens 100 % gestreckt worden sind.

2. Gelochte Folie (30) nach Anspruch 1, bei welcher die Mikrolöcher (37) eine Querschnittsfläche im Bereich von 0,019 x $10^{-6}$ $cm^2$ (0,003 tausendstel Zoll zum Quadrat) bis 2580 x $10^{-6}$ $cm^2$ (400 tausendstel Zoll zum Quadrat) haben.

3. Gelochte Folie (30) nach Anspruch 1 oder 2, bei welcher die Mikrolöcher (37) eine relative Streuung des Formfaktors von mindestens 25 % haben.

4. Gelochte Folie (30) nach einem der Ansprüche 1 bis 3, bei welcher die Mikrolöcher (37) einen äquivalenten hydraulischen Durchmesser von 1,27 x $10^{-3}$ cm (0,5 tausendstel Zoll) bis 63,5 x $10^{-3}$ cm (25 tausendstel Zoll) haben.

5. Gelochte Folie (30) nach einem der Ansprüche 1 bis 4, bei welcher die Mikrolöcher (37) eine relative Streuung des äquivalenten hydraulischen Durchmessers von mindestens 50 % haben.

6. Gelochte Folie (30) nach einem der Ansprüche 1 bis 5, welche eine Öffnungsfläche von 1 % bis 15 % hat, wobei diese Öffnungfläche durch die Mikrolöcher (37) realisiert ist.

7. Gelochte Folie (30) nach einem der Ansprüche 1 bis 6, bei welcher die faserartigen Elemente eine Länge im Bereich von 0,013 cm (0,005 Zoll) bis 0,127 cm (0,05 Zoll) haben.

8. Gelochte Folie (30) nach einem der Ansprüche 1 bis 7, bei welcher die faserartigen Elemente eine Breite im Bereich von 0,003 cm (0,001 Zoll) bis 0,089 cm (0,035 Zoll) haben.

9. Gelochte Folie (30) nach einem der Ansprüche 1 bis 8, bei welcher die faserartigen Elemente eine Dicke im Bereich von 0,0006 cm (0,0025 Zoll) bis 0,005 cm (0,002 Zoll) haben.

10. Gelochte Folie (30) nach einem der Ansprüche 1 bis 9, bei welcher die Mikrolöcher (37) in einem Muster getrennter Gruppen angeordnet sind.

11. Gelochte Folie (30) nach einem der Ansprüche 1 bis 9, bei welcher die Mikrolöcher (37) eine unregelmäßige Form haben und in den gelochten Bereichen der Folie zufällig verteilt sind.

12. Gelochte Folie (30) nach einem der Ansprüche 1 bis 11, welche weiterhin eine Vielzahl von Sekundäröffnungen (42) aufweist, deren Querschnittsflächen wesentlich größer sind als die Querschnittsflächen der Mikrolöcher (37).

13. Gelochte Folie (30) nach einem der Ansprüche 1 bis 12 mit einer Reihe paralleler Erhebungen (32) und einer Reihe von Tälern (33) zwischen der Reihe von Erhebungen (32), wobei eine jede Erhebung (32) durch ein Paar allgemein senkrecht gerichteter Seitenwände (31) gebildet wird, die sich an der Oberseite der Folie treffen und wobei die Täler (33) eine Vielzahl von Mikrolöchern (37) aufweisen, welche durch ein Netzwerk faserartiger Elemente (38) begrenzt sind.

14. Gelochte Folie (30) nach Anspruch 13, bei welcher die unteren Bereiche der abgewinkelten Seitenwände (31) eine Vielzahl von Mikrolöchern (37) aufweisen.

15. Gelochte Folie (30) nach Anspruch 13 oder 14, bei welcher die Erhebungen (32) in der Draufsicht eine sinuskurvenförmige Gestalt haben.

16. Gelochte Folie (40) nach einem der Ansprüche 1 bis 12 mit einer Vielzahl allgemein senkrecht ausgerichteter, durch schräge Seitenwände (41a) begrenzter kegelartiger Strukturen (41), wobei die schrägen Seitenwände (41a) eine Vielzahl von Tälern (43) zwischen den kegelartigen Strukturen (41) bilden und die Täler (43) eine Vielzahl von Mikrolöchern (37) aufweisen, welche durch ein Netzwerk faserartiger Elemente (38) begrenzt sind.

17. Gelochte Folie (40) nach Anspruch 16, bei welcher die unteren Bereiche der schrägen Seitenwände (41a) eine Vielzahl von Mikrolöchern (47) aufweisen.

18. Gelochte Folie (40) nach einem der Ansprüche 16 oder 17, bei welcher die kegelartigen Strukturen (41) Sekundäröffnungen (42) aufweisen, deren Querschnittsflächen wesentlich größer sind als die Querschnittsflächen der Mikrolöcher (47).

19. Gelochte Folie (50) nach einem der Ansprüche 1 bis 9 mit Mikrolöchern (57), die in einer Vielzahl von Gruppen (55) von Mikrolöchern (57) angeordnet sind.

20. Gelochte Folie (50) nach Anspruch 19, bei welcher jede Gruppe (55) mindestens eine Reihe (56) von Mikrolöchern (57) umfaßt, wobei diese Reihe (56) von Mikrolöchern (57) zu beiden Seiten einen Streifen ungelochten Materials aufweist.

21. Gelochte Folie (50) nach Anspruch 19 oder 20, bei welcher jede Gruppe (55) eine Vielzahl von Reihen (56) von Mikrolöchern (57) umfaßt, wobei benachbarte Reihen (56) von Mikrolöchern (57) durch Streifen (59) ungelochten Materials voneinander getrennt sind.

22. Gelochte Folie (50) nach einem der Ansprüche 19 bis 21, bei welcher die Bereiche (52) rund um die Gruppen (55) sowie zwischen benachbarten Gruppen (55) von Mikrolöchern (57) im wesentlichen frei von Mikrolöchern sind.

23. Absorbierendes Erzeugnis aus einem absorbierenden Kern, der mit einer gelochten Folie abgedeckt ist, wobei die gelochte Folie einem der Ansprüche 1 bis 22 entspricht.

24. Absorbierendes Erzeugnis nach Anspruch 23, welches ein Wundverband ist.

25. Absorbierendes Erzeugnis nach Anspruch 23 oder 24, bei welchem die beiden Hauptflächen des absorbierenden Kerns mit der gelochten Folie argedeckt sind.

26. Absorbierendes Erzeugnis nach Anspruch 23, welches eine Wegwerf-Windel ist.

27. Absorbierendes Erzeugnis nach Anspruch 23, welches eine Damenbinde ist.

28. Absorbierendes Erzeugnis nach Anspruch 23, welches weiterhin eine flüssigkeitsundurchlässige Rückseitenschicht aufweist, die angrenzend an eine Hauptfläche des absorbierenden Kernes angeordnet ist.

29. Damenbinde bestehend aus einem absorbierenden Kern mit einer oberen Hauptfläche und einer unteren Hauptfläche, einer flüssigkeitundurchlässigen Barriereschicht und einem Abdeckmaterial, wobei das Abdeckmaterial mindestens die obere Hauptfläche des absorbierenden Kernes berührt, das Abdeckmaterial eine gelochte Folie nach einem der Ansprüche 1 bis 22 ist und die Barriereschicht an der unteren Hauptfläche des absorbierenden Kernes angeordnet ist.

**30.** Damenbinde nach Anspruch 29, bei welcher mindestens 75 % der Mikrolöcher eine Querschnittsfläche von weniger als 2580 x $10^{-6}$ cm$^2$ (400 tausendstel Zoll zum Quadrat) haben.

**31.** Verfahren zum Herstellen einer gelochten Folie aus einem Polymermaterial mit einer Vielzahl von Mikrolöchern mit den Schritten:

a) Bereitstellen einer Startfolie (67) aus Polymermaterial mit einer Oberseite und einer Unterseite;
b) Bereitstellen einer Auflage (64) mit Auflagebereichen (74) an bestimmten Stellen zum Auflegen einer Folie (67), mit vertieften Zonen, in welche hinein die Folie durch das Anlegen von Fluidkräften verformt werden kann sowie mit Einrichtungen (80), welche das Ableiten des verwendeten Fluids von der Auflage ermöglichen;
c) Auflegen der Startfolie (67) auf die Auflage (64), wobei die Unterseite der Folie die Auflagefläche der Auflage berührt und die Oberseite von der Auflage abgewandt ist;
d) Richten einer Fluidkraft gegen eine Kontaktzone der Oberseite der Startfolie (67) mit einem Druck, der zur Bildung einer Vielzahl von Mikrolöchern ausreicht;
e) Bewegen der Folie (67) aus der Kontaktzone heraus und
f) Entfernen der nunmehr gelochten Folie (68) von der Auflage.

dadurch gekennzeichnet, daß

a) die bereitgestellte Startfolie (67) aus einem dehnbaren thermoplastischen Polymermaterial besteht und
d) die Fluidkraft in Gestalt säulenförmiger Strömungen auf das thermoplastische Polymermaterial gerichtet wird, um die Bildung einer Vielzahl von Mikrolöchern zu veranlassen und das Polymermaterial im Vergleich zu angrenzenden Bereichen der Startfolie, die während der Bearbeitung nicht der Lochung unterlagen, um mindestens 100 % zu strecken, um ein Netzwerk faserartiger Elemente zu erzeugen, welche die Mikrolöcher begrenzen.

**32.** Verfahren nach Anspruch 31, bei welchem das Fluid unter einem Druck im Bereich von 351 550 kgm$^{-2}$ (500psig) bis 1 124 960 kgm$^{-2}$ (1600psig) steht.

**33.** Verfahren nach Anspruch 31 oder 32, bei welchem das Fluid, welches auf die Oberseite der Folie gerichtet wird, Wasser mit einer Temperatur von mindestens 32,2 °C (90 °F) ist.

## Revendications

**1.** Film poreux comprenant un matériau polymérique ayant de multiples microtrous, caractérisé en ce que le matériau polymérique comprend un matériau polymérique thermoplastique étirable et en ce que les microtrous (37) sont définis par un réseau d'éléments fibreux (38), dans lequel le matériau polymérique thermoplastique comprenant lesdits éléments fibreux (38) a été étiré d'au moins 100 pour cent en comparaison avec des régions adjacentes du film de départ sur lesquelles on n'a pas effectué de perforation au cours du traitement.

**2.** Film poreux (30) selon la revendication 1, dans lequel lesdits microtrous (37) ont une aire dans la gamme de 0,019 x $10^{-6}$ cm$^2$ (0,003 mils carrés) à 2580 x $10^{-6}$ cm$^2$ (400 mils carrés).

**3.** Film poreux (30) selon la revendication 1 ou la revendication 2, dans lequel les microtrous (37) ont un coefficient de variation de facteur de forme d'au moins environ 25%.

**4.** Film poreux (30) selon l'une quelconque des revendications 1 à 3, dans lequel les microtrous (37) ont un diamètre hydraulique équivalent (EHD) dans la gamme de 1,27 x $10^{-3}$ cm (0,5 mil) à 63,5 x $10^{-3}$ cm (25 mils).

**5.** Film poreux (30) selon l'une quelconque des revendications 1 à 4, dans lequel les microtrous (37) ont un coefficient de variation de EHD d'au moins environ 50%.

**6.** Film poreux (30) selon l'une quelconque des revendications 1 à 5 ayant une aire ouverte comprise entre 1% et 15%, ladite aire ouverte étant créée par lesdits microtrous (37).

7. Film poreux (30) selon l'une quelconque des revendications 1 à 6, dans lequel les éléments fibreux (38) ont des longueurs dans la gamme de 0,013 cm (0,005 pouce) à 0,127 cm (0,05 pouce).

8. Film poreux (30) selon l'une quelconque des revendications 1 à 7, dans lequel les éléments fibreux (38) ont des largeurs dans la gamme de 0,003 cm (0,001 pouce) à 0,089 cm (0,035 pouce).

9. Film poreux (30) selon l'une quelconque des revendications 1 à 8, dans lequel les éléments fibreux (38) ont des épaisseurs dans la gamme de 0,0006 cm (0,00025 pouce) à 0,005 cm (0,002 pouce).

10. Film poreux (30) selon l'une quelconque des revendications 1 à 9, dans lequel lesdits microtrous (38) sont disposés en un motif de groupes distincts.

11. Film poreux (30) selon l'une quelconque des revendications 1 à 9, dans lequel lesdits microtrous (38) ont des formes irrégulières et sont répartis de manière aléatoire dans les régions poreuses du film.

12. Film poreux (30) selon l'une quelconque des revendications 1 à 11, comprenant en outre une multiplicité d'ouvertures secondaires (42) dont les aires sont substantiellement plus grandes que les aires desdits microtrous (38).

13. Film poreux (30) selon l'une quelconque des revendications 1 à 12, comprenant une série de sillons parallèles (32) et une série de vallées (33) entre ladite série de sillons, chacun desdits sillons étant formé par une paire de parois latérales (31) généralement orientées verticalement, qui se rencontrent à la surface supérieure dudit film, lesdites vallées comprenant la multiplicité de microtrous (37) définis par le réseau d'éléments fibreux (38).

14. Film poreux (30) selon la revendication 13 dans lequel les rangées inférieures desdites parois latérales (31) formant un angle comprennent une multiplicité desdits microtrous (37).

15. Film poreux (30) selon la revendication 13 ou la revendication 14, dans lequel lesdits sillons (32), en vue en plan, ont une configuration sinusoïdale.

16. Film poreux (30) selon l'une quelconque des revendications 1 à 12 comprenant une multiplicité de structures en forme de cône (41) généralement orientées verticalement définies par des parois latérales inclinées (41), lesdites parois latérales inclinées définissant une multiplicité de vallées (43) entre lesdites structures en forme de cône (41), lesdites vallées (43) comprenant la multiplicité de microtrous (47) définie par le réseau d'éléments fibreux (48).

17. Film poreux (30) selon la revendication 16, dans lequel les rangées inférieures desdites parois latérales (41a) inclinées comprennent une multiplicité desdits microtrous (47).

18. Film poreux (30) selon la revendication 16 ou la revendication 17, dans lequel les structures en forme de cône (41) comprennent des ouvertures secondaires dont les aires sont substantiellement plus grandes que les aires desdits microtrous (47).

19. Film poreux (50) selon l'une quelconque des revendications 1 à 9 comprenant lesdits microtrous (57) étant disposés en une multiplicité d'agglomérats (55) de microtrous.

20. Film poreux (50) selon la revendication 19, dans lequel chacun desdits agglomérats (55) comprend au moins une rangée (56) desdits microtrous (57), ladite rangée (56) de microtrous (57) ayant une bande de matériau sans ouverture de chaque côté de celle-ci.

21. Film poreux (50) selon la revendication 19 ou la revendication 20, dans lequel chacun desdits agglomérats (55) comprend une multiplicité de rangées (56) desdits microtrous (57) et les rangées adjacentes (56) sont séparées les unes des autres par des bandes (59) de matériau sans ouverture.

22. Film poreux (50) selon l'une quelconque des revendications 19 à 21, dans lequel les régions (50) autour et entre les agglomérats voisins (55) de microtrous (57) sont essentiellement dénuées de microtrous.

23. Produit absorbant comprenant une partie centrale absorbante recouverte avec un film poreux, ledit film poreux étant selon l'une quelconque des revendications 1 à 22.

**24.** Produit absorbant selon la revendication 23 qui est un pansement pour blessures.

**25.** Produit absorbant selon la revendication 23 ou la revendication 24, dans lequel les deux surfaces principales de ladite partie centrale absorbante sont recouvertes avec ledit film poreux.

**26.** Produit absorbant selon la revendication 23 qui est une couche jetable.

**27.** Produit absorbant selon la revendication 23 qui est une serviette hygiénique.

**28.** Produit absorbant selon la revendication 23 comprenant de plus une couche dorsale imperméable aux liquides située contre une surface principale de ladite surface absorbante.

**29.** Serviette hygiénique comprenant une partie centrale absorbante ayant une surface principale supérieure et une surface principale inférieure; une couche barrière imperméable aux liquides; et un matériau protecteur, ledit matériau protecteur étant en contact avec au moins la surface supérieure de ladite partie centrale absorbante, ledit matériau protecteur comprenant un film poreux selon l'une quelconque des revendications 1 à 22, et ladite couche barrière étant placée adjacente à la surface inférieure de ladite partie centrale absorbante.

**30.** Serviette hygiénique selon la revendication 29, dans laquelle au moins 75% des microtrous ont des aires inférieures à 400 mils carrés.

**31.** Procédé de formage d'un film poreux comprenant un matériau polymérique comprenant une multiplicité de microtrous, ledit procédé comprenant les étapes consistant :

a) à prendre un film de départ (67) comprenant un matériau polymérique et ayant une surface supérieure et une surface inférieure:
b) à prendre un élément dorsal (64) comprenant des régions support localisées (74) pour soutenir un film (67), des zones évidées dans lesquelles le film peut être déformé par l'application sur celui-ci de forces hydrauliques, et un dispositif (80) pour permettre d'évacuer ledit fluide appliqué dudit élément dorsal;
c) à faire supporter ledit film de départ (67) sur ledit élément dorsal (64) avec la surface inférieure dudit film étant en contact avec la surface support dudit élément dorsal et avec la surface supérieure dudit film faisant face à l'extérieur dudit élément dorsal.
d) à diriger une force hydraulique contre la surface supérieure dudit film de départ (67) dans une zone de contact et à une pression suffisante pour provoquer la formation d'une multiplicité desdits microtrous.
e) à déplacer ledit film (67) de ladite zone de contact; et
f) à retirer ledit film maintenant poreux (68) dudit élément dorsal,

caractérisé par :

(a) la fourniture d'un film de départ (67) comprenant un matériau polymère thermoplastique extensible; et
(b) l'envoi d'une force hydraulique sous la forme de courants colonnaires pour provoquer la formation d'une multiplicité de microtrous et pour étirer le matériau thermoplastique polymérique d'au moins 100 pour cent en comparaison avec les régions adjacentes du film de départ sur lesquelles on n'a pas effectué de perforation au cours du traitement de façon à obtenir un réseau d'éléments fibreux définissant lesdits microtrous.

**32.** Procédé selon la revendication 31, dans lequel ledit fluide est sous une pression dans la gamme de 351550 kgm$^{-2}$ (550 psig) à 1124960 kgm$^{-2}$ (1600 psig).

**33.** Procédé selon la revendication 31 ou la revendication 32, dans lequel ledit fluide est l'eau qui est dirigée à la surface supérieure dudit film à une température d'au moins environ 32,2°C (90°F).

FIG. 1

*FIG. 2*

*FIG. 3*

FIG. 4

FIG. 5

FIG. 6

FIG.7

FIG. 8

FIG. 9

FIG. 10

FIG. II

58     *FIG. 12*     57

*FIG.13*

FIG. 14

FIG. 15

## FIG. 16

FIG.17

## FIG. 18

## FIG. 19

BOX 1 — POSITION FILM
ONTO SUPPORT
MEMBER

↓

BOX 2 — PASS SUPPORT
MEMBER UNDER
HIGH PRESSURE
WATER JETS

→ BOX 3

SUCTION WATER
AWAY UNDER
SUPPORT MEMBER

↓

BOX 4 — DE-WATER
APERTURED FILM
VIA SUCTION

↓

BOX 5 — REMOVE APERTURED
FILM FROM SUPPORT
MEMBER

↓

BOX 6 — REMOVE
RESIDUAL
WATER

→ ROLL UP — BOX 7

*FIG. 20*

**FIG. 21**

67
93
95
96a
99
91
96
96
98
99
99a
92
97
96f
90
68

FIG. 22

FIG. 23

FIG. 24

## FIG. 25

## FIG. 26

*FIG. 27*

200

208

28

206

28

214

210

206

202

206

214

204

212

*FIG. 28*